(19) **Europäisches Patentamt / European Patent Office / Office européen des brevets**

(11) **EP 2 641 966 A1**

(12) **EUROPEAN PATENT APPLICATION**
published in accordance with Art. 153(4) EPC

(43) Date of publication:
**25.09.2013 Bulletin 2013/39**

(51) Int Cl.:
***C12N 5/07*** (2010.01)

(21) Application number: **11841159.4**

(22) Date of filing: **18.11.2011**

(86) International application number:
**PCT/JP2011/076711**

(87) International publication number:
**WO 2012/067240 (24.05.2012 Gazette 2012/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2010 JP 2010259525**

(71) Applicant: **Seiren Kabushiki Kaisha**
**Fukui-shi**
**Fukui 918-8560 (JP)**

(72) Inventors:
• **KUNITOMI, Yoshihiro**
  **Fukui-shi**
  **Fukui 918-8560 (JP)**
• **SASAKI, Masahiro**
  **Fukui-shi**
  **Fukui 918-8560 (JP)**

(74) Representative: **Regimbeau**
**20, rue de Chazelles**
**75847 Paris Cedex 17 (FR)**

(54) **VITRIFICATED STORAGE SOLUTION FOR CELLS**

(57) The vitrification medium for cells according to the present invention is a vitrification medium for cells comprising a cell membrane permeable substance and a cell membrane non-permeable substance, in which, the content of the cell membrane permeable substance is in the range of 30 to 50% by volume, and the osmotic pressure generated in association with the cell membrane non-permeable substance, which is a fraction of the total osmotic pressure on the cell membrane on suspending the cells in the vitrification medium, is in the range of 280 mOsm or more. The vitrification medium for cells according to the present invention is excellent in operability and safety.

EP 2 641 966 A1

**Description**

REFERENCE TO THE RELATED APPLICATION

The present application is based on the prior Japanese Patent Application No. 2010-259525 filed on November 19, 2010 and claims the advantages of its priority, the content of which is incorporated herein by reference in their entirety.

BACKGROUND OF THE INVENTION

FIELD OF THE INVENTION

The present invention relates to a storage solution for vitrifying and cryopreserving cells. Particularly, the present invention relates to a vitrification medium for cells (or vitrification storage solution for cells), which scarcely causes the lowering of the survival rate of the cells due to operation times and may be suitably used for primate ES cells or primate iPS cells.

RELATED ARTS

The cryopreservation of cells has hitherto been conducted routinely for the purpose of preventing the cultured cells from denaturation due to passages and contamination of saprophytic bacteria and utilizing the cells for a long period. Direct freezing of cells will form ice crystals in and out of the cells, which are physically damaged and led to death. Particularly, a number of structures vital to biological activity are present in cells, and the formation of the ice crystals within the cells is fatal. A storage solution containing a cell-membrane-permeable cryoprotective agent such as dimethyl sulfoxide (DMSO) is required for preventing the damage of cells, and a storage solution excellent in the storage efficiency is earnestly desired to be developed.

In general, the cryopreservation methods of cells are broadly classified into a slow freezing method and a vitrification method.

The term "slow freezing method" refers to a method in which the cells are suspended in a storage solution having about 5 to 20% by volume of a cryoprotective agent added to a culture medium or serum, and after the infiltration of the cryoprotective agent into the cells, temperature is controllably lowered gradually in order to finally preserve the cells in the liquid nitrogen (-196°C).

Cooling of the suspension containing cells leads to the supercooling condition of both intracellular and extracellular fluids. The cells are released from the supercooling condition at a certain temperature and ice crystals are formed. The temperature is determined depending on the concentration of the solutions and the type of core materials, and generally the temperature at which ice crystals are formed is higher in the extracellular fluid than in the intracellular fluid.

Meanwhile, when the suspension is cooled at a higher cooling rate, the intracellular and extracellular fluids get out of the supercooling condition almost at the same time and a large amount of ice crystals are formed both in and out of the cells, which undergo physical damage.

In contrast, if the suspension is cooled at a moderate rate, ice crystals are first formed extracellularly. Because of freezing of water alone, the extracellular fluid is gradually concentrated and becomes hypertonic. As a result, water within the cells moves outward from the interior of the cells due to the osmotic pressure difference between the intracellular and extracellular fluids. This is so called the "dehydration" phenomenon. This dehydration results in the increase in the concentration of the intracellular fluid containing the cryoprotective agent, and thus the intracellular fluid becomes vitrified.

On the other hand, when the suspension is cooled at a low  rate, the excessive growth of ice crystals formed in the exterior of the cells may cause the physical damage of the cells or the prolonged hypershrinkage condition associated with the dehydration may damage organelle.

The expression "vitrification" is described herein.
Liquid is comprehended as a condition that thermal oscillation surpasses intermolecular forces and particles within a phase can move freely. When a liquid substance is cooled, thermal oscillation falls below intermolecular forces at a certain temperature and particles within the phase will lose fluidity. At this temperature, the particles are rearranged into an energetically stable crystal structure according to some substances. For instance, water is ordinarily crystalized into ice at a low temperature of 0°C or less. However, the viscosity of the liquid is increased without crystallization even at lower temperatures depending on the external factors such as pressure and cooling rate in some substances, which are solidified directly. This phenomenon is designated as vitrification. Substances to be vitrified include, for example, glass comprising silicon dioxide as the main ingredient, which is known as a typical glass material.

Vitrification is largely influenced by cooling rate, and generally the higher the cooling rate, the more easily the liquid is vitrified. The temperature at which the liquid is vitrified is called the glass transition temperature, which varies depending on the types of substances or the cooling rates. It is known that water may also be vitrified depending on conditions and is easily vitrified by mixing it with an easily vitrified material such as glycerin.

In the slow freezing method, the intracellular fluid is vitrified for suppressing the formation of ice crystals, so that a survival rate is maintained in a practical level even after melting. However, it is known that the cells which can be efficiently preserved by the slow freezing method are limited to a few cells such as established cell lines and that some of the primary cell culture, the normal cells, the germ cells, and the embryonic stem cells (ES cells) are difficult to be preserved efficiently. In addition, large differences between species were also found, and, for example, there was a problem that while a relatively high survival rate was exhibited in the ES cell of rodents including mice, any satisfactory survival rates were not found in the ES cells of primates such as human.

The term "vitrification method" refers to the method that the vitrification is applied more positively to the preservation of cells. This method has been used in many cases for preserving ovules, fertilized eggs and embryos which are difficult to preserve by the slow freezing method. The vitrification method comprises suspending cells in a vitrification medium which contains a cryoprotective agent in about 30 to 50% by volume, adding the suspension to liquid nitrogen, and rapidly cooling it to or below the glass transition temperature to vitrificate both interior and exterior of the cell. The term "rapid freezing method" is often used in the sense of comparing the vitrification method with the slow freezing method described above. The storage solution used in the vitrification method (i.e., vitrification medium) is often referred to as VS (vitrification solution).

Similarly, it is contemplated also in the slow freezing method that the intracellular fluid is concentrated as a result of dehydration and finally vitrified. However, it is contemplated in the slow freezing method that fine ice crystals formed extracellularly may damage cells. In addition, in cells having a high water content such as ovules, fertilized eggs and embryos, it is contemplated that intracellular dehydration remains incomplete and thus ice crystals may also be formed intracellularly.

On the contrary, in the vitrification method, the extracellular fluid, that is, the storage solution is vitrified without forming ice crystals owing to a high concentration of a cryoprotective agent. Also, the intracellular fluid is directly dehydrated by the hyperosmotic storage solution, replaced with the cryoprotective agent, and vitrified.

As described above, the vitrification method has been developed for cells and the like which are difficult to be preserved efficiently by the slow freezing method and exploited industrially for storing bovine in vitro fertilization embryo in the livestock industry. In the investigation level, improvements have been carried out with mouse or rat embryos. At the present time, the vitrification medium used in the vitrification method of embryos includes typically "DAP213" which contains dimethyl sulfoxide, acetamide and propylene glycol in the concentrations of 2 M, 1 M and 3 M, respectively.

While a plurality of methods are known as the vitrification method of embryos with use of DAP213, the following procedures are involved in most of cases:

(1) equilibration of embryo in stages with a solution containing a low concentration of a protective agent;
(2) vitrification of embryo by rapid cooling with liquid nitrogen;
(3) melting by the addition of a warmed diluent;
(4) the embryo is transferred to culture after washing.

The term "equilibration" herein means to leave cells or the like standing until the apparent movement of a cell membrane permeable substance such as water or the like through a cell membrane or the channels or pores on the membrane disappears in the interior or exterior of the cells. The time required for the "equilibration" depends on cell types and the like, and, for instance, it is usually about 1 to 5 minutes in mouse embryo.

Recently, the vitrification method has been utilized also in the preservation of primate ES cells in addition to ovules, fertilized eggs, embryos, and the like. Furthermore, induced pluripotent stem cells (iPS cells) having the virtually same properties as the ES cells have been developed, and human iPS cells are also preserved generally by the vitrification method. In addition, DAP213 is also extensively used as the vitrification medium of the primate ES/iPS cells.

The method for preparing DAP213 and the vitrification method of the primate ES/iPS cells with DAP213 have been disclosed in the home page of Riken Center for Developmental Biology, Incorporated Administrative Agency (Riken CDB) (http://www.cdb.riken.go.jp/). The vitrification method with DAP213 has been established as the standard preservation method of primate ES/iPS cells in Japan which is readily operated and has a high survival rate, and research facilities such as Riken CDB and Riken BioResource Center, Incorporated Administrative Agency (Riken BRC) recommend the vitrification method as the preservation method of primate ES/iPS cells.

International Publication WO 2005/045007 discloses the preservation method of primate ES cells with a storage medium containing dimethyl sulfoxide (DMSO), propylene glycol and a culture medium, preferably acetamide. It is considered in the specification described above that primate ES cells can be efficiently preserved by using a freezing medium for primate ES cells. In this connection, the medium disclosed herein is available as a prepared vitrification medium (trade name "freezing medium for primate ES cells") from Reprocell Inc.

However, although the expression "vitrification method with DAP213" is used in a lump, it is different in details between the cases of targeting primate ES/iPS cells and of targetting ovules, fertilized eggs and embryos. For instance, when primate ES/iPS cells are targeted, equilibration prior to vitrification which is carried out in the case of targetting ovules, fertilized eggs and embryos is not usually required.

Thus, while the vitrification method with DAP213 has been establishing as a standard preservation method for primate ES/iPS cells, it is still desired to be improved in several points.

One of the problems is operability. That is, the time required for a series of operations, particularly the time required from suspending the cells in DAP213 to pouring the suspension into liquid nitrogen seriously affects the survival rate of the cells in the vitrification method.

By way of example, it has been described by Fujioka et al. that in an experiment with simian ES cells, the survival rate is decreased from 100%, in which the time required from suspending the cells in DAP213 to pouring the suspension into liquid nitrogen is not longer than 15 seconds, to 30% or less when the required time is 30 seconds and to 10% or less when the time is 60 seconds (A simple and efficient cryopreservation method for primate embryonic stem cells, Fujioka T, Yasuchika K, Nakamura Y, Nakatsuji N, Suemori H. Int J Dev Biol 48:1149-54, 2004).

Namely, in the vitrification method for primate ES/iPS cells with DAP213, extremely rapid operations are required and thus skilled technique is necessary. This is apprehensive of widely varying in results depending on the skills of operators, so that a stable result is difficult to be obtained.

Another point to be improved is the problem of safety. DAP213 contains acetamide, which is the substance suspected of carcinogenicity. Acetamide is not always suitable for use as an ingredient of a storage solution to store cells for regenerative medicine.

International Publication WO 2007/058308 discloses an aqueous solution for storing cells which contains a thickener, a cryoprotective agent, a saccharide, no natural ingredients derived from animals and preferably a phosphate buffer, and preferably maintains an osmotic pressure in the range of 1000 mOsm or more. However, the aqueous solution for storing cells herein means the so-called storage solution by the slow freezing method, which is different from the vitrification medium. In addition, the osmotic pressure herein refers to the total osmotic pressure.

Japanese Patent Laid-Open Publication No. 2001-502664 (International Publication WO 98/09514) and Japanese Patent Laid-Open Publication No. 2001-517204 (International Publication WO 97/45010) disclose the methods for preserving cells by vitrification, in which a preservation solution containing a permeable cryoprotective agent, a non-permeable cryoprotective agent, a non-permeable co-solute is used. However, it is emphasized in the specification that dehydration should be conducted by a method which will not decrease the cell volume since the shrinking of cells is harmful to the preservation of the cells, and thus it is regarded that the shrinking of cells should be avoided as far as possible.

Japanese Patent No. 3694730 discloses a cooling storage solution comprising at least one cell membrane permeable substance selected from methanol or ethanol, at least one substance having an ability for suppressing the formation of ice crystals which is selected from the group consisting of dimethyl sulfoxide, ethylene glycol, propylene glycol, 1,3-butane diol, 2,3-butane diol and glycerol, and at least one cell membrane non-permeable dehydration promoting substance selected from the group consisting of sucrose, trehalose, raffinose, lactose and fructose (Claim 1 of the specification). However, this storage solution primarily aims at tissues and organs, particularly genital organs and contains methanol or ethanol as the essential ingredients.

SUMMARY OF THE INVENTION

The present inventors have now successfully maintained the survival rate of cells in a high level with a vitrification medium comprising a cell membrane permeable substance and a cell membrane non-permeable substance by adjusting the osmotic pressure generated in association with the cell membrane non-permeable substance, which is a fraction of the total osmotic pressure generating in the cell membrane upon suspending the cells in the vitrification medium, to 280 mOsm or more, even if the operation comprising the steps from suspending required cells in a storage solution to cryopreservation is not conducted in a short time of, for example, 15 seconds or less as in the case of DAP213. Thus, skillfulness required for the operation of preservation treatment by vitrification can be lowered, and it has become possible to desire a stable result by reducing the dispersion due to the technical levels of operators. Furthermore, the vitrification medium is excellent in safety, because it contains no acetamide suspected of carcinogenicity. The present invention is based on such information.

Thus, the object of the present invention is to provide a vitrification medium for cells that is excellent in operability and safety.

The vitrification medium for cells according to the present invention is a vitrification medium for cells comprising a cell membrane permeable substance and a cell membrane non-permeable substance, in which

the content of the cell membrane permeable substance is in the range of 30 to 50% by volume, and

the osmotic pressure generated in association with the cell membrane non-permeable substance, which is a fraction of the total osmotic pressure on the cell membrane on suspending the cells in the vitrification medium, is in the range of 280 mOsm or more.

According to a preferred embodiment of the present invention, the cell membrane permeable substance in the vitrification medium of the present invention is one or more selected from the group consisting of ethylene glycol (EG), propylene glycol (PG), 1,3-propanediol (1,3-PD), butylene glycol (BG), isoprene glycol (IPG), dipropylene glycol (DPG), glycerin and dimethyl sulfoxide (DMSO).

According to a more preferred embodiment of the present invention, the cell membrane permeable substance in the vitrification medium of the present invention is one or two selected from the group consisting of ethylene glycol (EG), propylene glycol (PG) and dimethyl sulfoxide (DMSO).

According to another preferred embodiment of the present invention, the cell membrane non-permeable substance in the vitrification medium of the present invention is one or more selected from the group consisting of sodium chloride, potassium chloride, disodium hydrogen phosphate, potassium dihydrogen phosphate, monosaccharides, disaccharides, polysaccharides, trisaccharides, sugar alcohols, Ficoll, polyethylene glycol, polyvinyl alcohol and polyvinylpyrrolidone.

According to another more preferred embodiment of the present invention, the cell membrane non-permeable substance in the vitrification medium of the present invention comprises a physiological environment adjusting substance selected from the group consisting of sodium chloride, potassium chloride, disodium hydrogen phosphate and potassium dihydrogen phosphate and a vitrification promoting substance selected from the group consisting of trehalose and sucrose.

According to another preferred embodiment of the present invention, the cell membrane non-permeable substance in the vitrification medium of the present invention comprises trehalose in the range of 0.2 M to 1 M as the concentration of the medium.

According to a preferred embodiment of the present invention, the vitrification medium for cells according to the present invention further comprises an ice crystal growth inhibitor selected from the group consisting of an antifreeze protein and sericin.

According to further preferred embodiment of the present invention, the vitrification medium of the present invention is used for the vitrification preservation of a pluripotent stem cell, more preferably for the vitrification preservation of primate ES cells or primate iPS cells (primate ES/iPS cells).

According to another embodiment of the present invention, there is provided a vitrification method for cells which comprises suspending cells in the vitrification medium for cells according to the present invention followed by rapidly cooling the vitrification medium containing the cells with liquid nitrogen for vitrification, in which the osmotic pressure generated in association with the cell membrane non-permeable substance, which is a fraction of the total osmotic pressure on the cell membrane on suspending the cells in the vitrification medium, is in the range of 280 mOsm or more.

According to the present invention, it is possible to provide a vitrification medium which is excellent in operability and safety and can efficiently preserve cells, particularly primate ES/iPS cells. Specifically, the take rate of ca. 60% could be obtained even if simian ES cells were suspended in the vitrification medium of the present invention and after 60 seconds the suspension was poured into liquid nitrogen (as described hereinafter, the "take rate" in place of the "survival rate" is used with respect to the preservation efficiency of simian ES cells. In this place, the take rate is calculated on the basis of the number of undifferentiated colonies having taken after three or four days from the seeding of the cells without vitrification as 100). Hitherto, when DAP213, which has been most commonly used, was used, it was important to pour the cell suspension into liquid nitrogen within 15 seconds after suspending the cells in DAP213 for avoiding the lowering of the survival rate. Due to this fact, skillfulness was required for operations and wide dispersion in results was apprehended to occur depending on the technical skills of operators, so that a stable result was not always obtained. As described above, the vitrification medium of the present invention requires no rapidity of operations unlike in the case with DAP213 and thus dispersion in results due to the technical levels of operators scarcely occurs, so that it has become possible to desire a stable result. Furthermore, the vitrification medium is excellent in safety, because it contains no acetamide suspected of carcinogenicity. Since the vitrification medium of the present invention is useful for the preservation of primate ES cells and primate iPS cells, it is extremely important that the use of acetamide suspected of carcinogenicity can be avoided in consideration of the applications to medicine.

BRIEF DESCRIPTION OF THE DRAWINGS

Figure 1 is a schematic diagram which illustrates the volume changes of cells suspended in the vitrification medium before vitrification.
Figure 2 is a schematic diagram which illustrates the amount changes of the cell membrane permeable substance after suspending cells in the vitrification medium.
Figure 3 is a schematic diagram which illustrates the volume changes of vitrified cells on melting.

Figure 4 is a graph which illustrates the survival rates of mouse ES cells after vitrification in Example 1.

Figure 5 is a graph which illustrates the survival rates of mouse ES cells after vitrification in Example 2.

Figure 6 is a graph which illustrates the results in Example 3.

Figure 7 is a graph which illustrates the influences of trehalose and osmotic pressures in Example 5 on the survival rates after vitrification.

Figure 8 is a graph which illustrates the influences of osmotic pressures for DAP in Example 6 on the survival rates after vitrification.

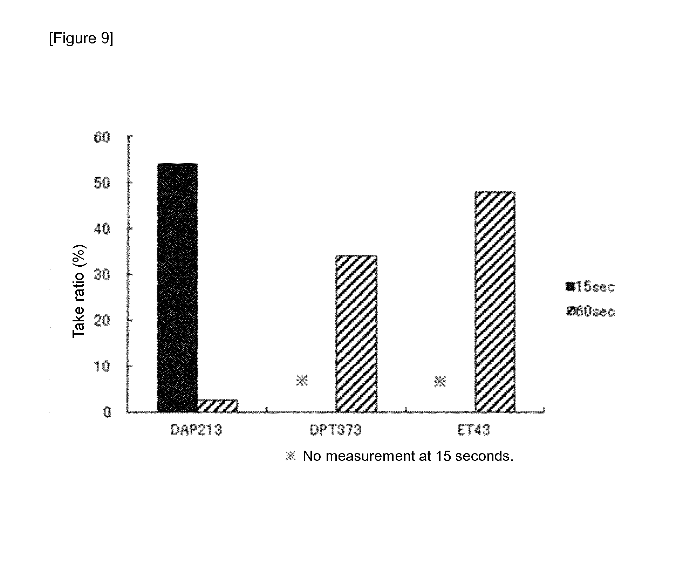

Figure 9 is a graph which illustrates the take rates of CMES in Example 7.

Figure 10 is a graph which illustrates the influences of trehalose and osmotic pressures in Example 7 on the survival rates after vitrification.

Figure 11 is a graph which illustrates the results of the efficiency evaluation of vitrification media for CMES cells in Example 7.

Figure 12 is a graph which illustrates the results of the examination of ice crystal growth inhibitors in Example 8.

## DETAILED DESCRIPTION OF THE INVENTION

### OSMOTIC PRESSURE

First, the relationship between the osmotic pressure and the amounts of substances in physical chemistry is briefly described.

Osmotic pressure ($\pi$) refers to the pressure applied to an ideal semipermeable membrane (a membrane which is permeable only to a solvent but not to a solute), on both sides of which are placed a solution and a pure solvent, and can be obtained from the equation of van't Hoff as follows:

$$\pi = \mathrm{MRT}$$

wherein M represents the volume molar concentration of a solute molecule or ion in a solution, R represents the gas constant and T represents the absolute temperature.

As is apparent from the equation, the osmotic pressure is in proportion to the volume molar concentration at a constant temperature. When the solute ionizes in a solvent such as the case of a salt, the volume molar concentration is estimated from the number of particles after ionization. The substance amount of a solute which brings about an osmotic pressure equivalent to the 1 M solution of a non-ionizable ideal solute is defined as 1 Osm, and generally Osm is also used as the unit of osmotic pressure.

In a sufficiently dilute aqueous solution, the difference of the volume of the solution and the volume of a solvent is so small to be neglected, and the volume molar concentration (number of moles of solute/total volume of solution) is equivalent to the weight molar concentration (number of moles of solute/weight of solvent alone). Thus, in order to find the osmotic pressure of a dilute aqueous solution, the weight molar concentration of a solute molecule or ion is calculated from the depression degree of freezing point (which is proportional to weight molar concentration) and is shown as the value with the unit Osm/kg which corresponds to the osmotic pressure. The true osmotic pressure calculated from the volume molar concentration is often expressed as the value with the unit Osm/L.

However, since the depression degree of freezing point is proportional to mass molar concentration and the osmotic pressure is proportional to volume molar concentration, the value of the osmotic pressure (having the unit Osm/kg) calculated from the depression degree of freezing point diverges from the value of the true osmotic pressure as the concentration of the solution increases.

Since the vitrification medium according to the present invention is a relatively concentrated solution containing 30 to 50% by volume of a cell membrane permeable substance (particularly, cryoprotective agent), the unit of osmotic pressure in this specification means Osm/L, which is often denoted as Osm for simplification.

Next, the influence of the osmotic pressure on cells is described.

The cell membrane freely permeates water but not polymers or ions and exhibits properties close to semipermeable membranes. Hence, if the concentration difference generates between the inside and outside solutions of cells, water moves due to the osmotic pressure.

In this specification, the solution is designated "isotonic" when apparent movement of water does not occur on dipping cells in a solution and the cell volume is not changed, the solution is designated "hypertonic" when the volume of the cells is reduced by the movement of water, and the solution is designated "hypotonic" when the volume of the cells is expanded by the movement of water.

In addition, a solution such as plasma having approximately the same osmotic pressure in the range of about 270 to 300 mOsm as that of the body fluid which fills the outside of cells is designated "an isotonic solution", and solutions having an osmotic pressure higher or lower than that of the isotonic solution are generally designated "a hypertonic solution" and "a hypotonic solution", respectively.

Generally, the isotonic solution is isotonic, the hypotonic solution is hypotonic and the hypertonic solution is hypertonic, but these relationships may be destroyed due to the presence of a cell membrane permeable substance in the solute. The difference of the osmotic pressure between the outside and inside of the cells in the presence of the cell membrane permeable substance is reduced together with the penetration of the cell membrane permeable substance into the cells, and no difference occurs at equilibrium. Thus, the volume of the cells at equilibrium will be determined by the concentration of the non- permeable substance in the extracellular fluid.

Since the membrane structure will be destroyed by the overload on the cell membrane due to the excessive concentration difference between the intracellular fluid and the extracellular fluid, biomaterials such as cells are generally treated within a solution isotonic to body fluid in vitro.

As described above, the isotonic solution refers to a solution having an osmotic pressure in the range of about 270 to 300 mOsm and is generally prepared as a solution containing a salt or a sugar as a main solute. For example, the isotonic solution is a ca. 0.9% by weight aqueous solution in the case of a physiological saline solution containing sodium chloride as a solute. The other isotonic solution used in the treatment of a biomaterial in vitro includes many well known isotonic solutions such as phosphate buffered saline (PBS) and Ringer's solution containing salts, salts, saccharides, amino acids or proteins. A culture medium used in cell culture has also been adjusted to be isotonic to the cell.

Experiments for dipping cells in solutions having various levels of osmotic pressure are known as a method for artificially causing the change of cell volume and the destruction of the cell membrane due to the difference of osmotic pressure.

When the cells are dipped into "the isotonic solution", the movement of water does not occur because of the equivalent osmotic pressure between the intracellular fluid and the extracellular fluid, and thus no change in the cell volume is observed.

When the cells are dipped into "the hypertonic solution", water moves from inside the cells to outside the cells due to the higher osmotic pressure in the extracellular fluid, and thus the cells will be shrunk. The shrink of the cells lasts until the osmotic pressure of the intracellular fluid is equivalent to that of the extracellular fluid by the condensation of the intracellular fluid or the shrink reaches the structural limitation of the cells.

When the cells are dipped into "the hypotonic solution", water moves from outside the cells to inside the cells due to the lower osmotic pressure in the extracellular fluid, and thus the cells will be expanded. In contrast with the case of shrink, the expansion of the cells lasts until the osmotic pressure of the intracellular fluid is equivalent to that of the extracellular fluid by the dilution of the intracellular fluid or the expansion reaches the structural limitation of the cells (e.g., support by the cell wall). When water moves into the cells over the structural limitation, the cell membrane is destroyed. Generally, as the animal cell has no cell wall, the cell membrane is apt to be damaged under the hypotonic environment.

While osmotic pressure is defined with an ideal semipermeable membrane (membrane which permeates the solvent alone but not the solute), the cell membrane is known to permeate monomeric substances in addition to water as a solvent. It is known that aquaporin which is a pore through which water permeates is present on the cell membrane. Hence, water has higher permeability than that of the other molecules. Whether a molecule can permeate the cell membrane or not is determined by the size of the molecule and the existence or not of the electric charges or polarities. While ions having electric charges cannot permeate the cell membrane by passive diffusion, monomeric substances having no electric charges easily permeate the cell membrane. Thus, these cell membrane permeable substances, which are different in the rate of diffusion due to the molecular weights or polarities, influence the volumes at equilibrium.

Therefore, as regards whether a solution is isotonic, hypertonic or hypotonic, these cell membrane permeable substances may be excluded. Thus, dimethyl sulfoxide, ethylene glycol, propylene glycol and the like, which are used as the cryoprotective agent, are generally considered not to influence the volume of the cells.

In addition, the term "freezing" sometimes means the formation of ice crystals and the vitrification method is defined not as the cryopreservation method but as the extremely low temperature preservation method, as the case may be, but the term "freezing" shall herein mean the change of a substance from liquid to solid by cooling.

On the other hand, the term "melting" shall herein mean the change of a substance from solid to liquid by heating.

## VITRIFICATION AND MELTING OF CELLS AND THE CHANGE OF CELL VOLUME

Based on the above description, the present inventors have examined how the volume of the cells changes in the vitrification of the cells. In this connection, the following description is a theory and is not intended to limit the present invention.

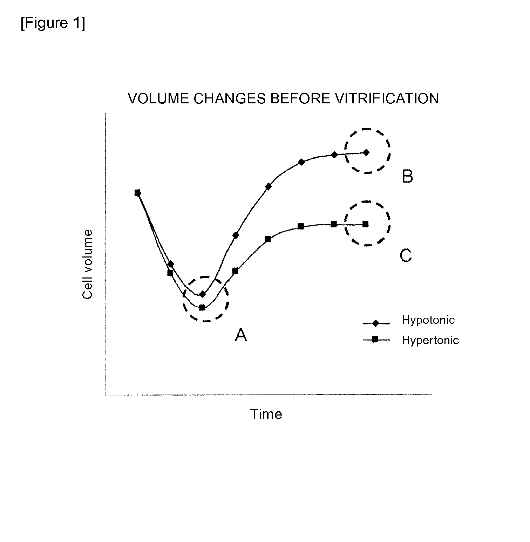

The change of cell volumes with the passage of time in the suspension of cells in a vitrification medium is considered as shown in Figure 1.

That is, when the cells are contacted with a vitrification medium having a high total osmotic pressure (containing a cell membrane permeable substance and a cell membrane non-permeable substance), the movement of water to the outside of the cells and the diffusion of a cell membrane permeable substance into the cells occur in the same time. Immediately after the contact of the cells and the vitrification medium, the velocity of water moving to outside the cells is generally higher in comparison with the velocity of the cell membrane permeable substance diffusing inside the cells, and thus the cells are predicated to be temporally shrunk (see the part "A" in Figure 1). In this case, this step is often called "the dehydration phase".

In this connection, the term "total osmotic pressure" herein refers to the osmotic pressure generating on the basis of the whole vitrification medium between inside and outside of the cell membrane of the cells in the suspension of the cells in the vitrification medium.

Thereafter, as the cell membrane permeable substance diffuses into the cells, the osmotic pressure within the cells increases and the volume of the cells increases (hereinafter, this step is often called "the diffusion phase"). Further, when the osmotic pressures of the cell membrane permeable substance of the inside and the outside of the cells become equivalent, the change of the cell volume stops (hereinafter, this step is often called "the equilibrium phase by the storage solution", or simply "the equilibrium phase"). The volume of the cells at equilibrium depends on the concentration of the cell membrane non-permeable substance in the extracellular fluid (storage solution), and the volume of the cells will be equivalent to the one in the physiological environment if the osmotic pressure of the cell membrane non-permeable substance in the extracellular fluid is "isotonic", the volume will be equivalent to the one in the expanding state (see the part "B" in Figure 1) if the osmotic pressure is "hypotonic", and the volume will be equivalent to the one in shrinking state if the osmotic pressure is "hypertonic" (see the part "C" in Figure 1). Since the volume of the cells is fixed at the time of vitrification, the cells, for example, vitrified in "the dehydration phase" is fixed at a state more shrunk than those in the ordinary physiological environment. On the other hand, the cells vitrified in "the equilibrium phase" will be able to be fixed at a state equivalent to the physiological environment, or at a shrunk or expanded state depending on the osmotic pressures of the cell membrane non-permeable substance in the storage solution.

Next, the amount of the cell membrane permeable substance present in the cells was examined.

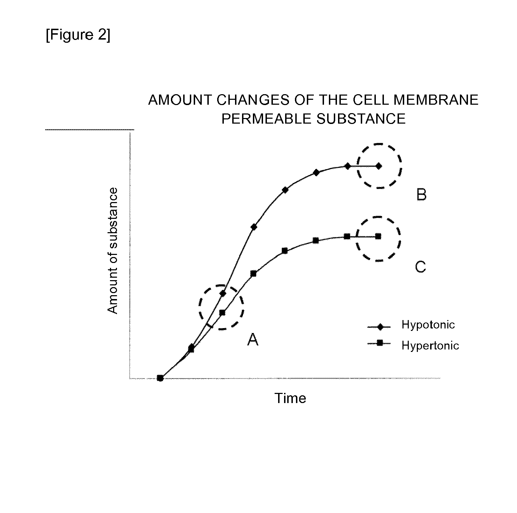

The amount of the cell membrane permeable substance within the cells increases along with the passage of time from suspending the cells in the storage solution to vitrification, and reached maximum at equilibrium (see Figure 2) . The amount of the cell membrane permeable substance at equilibrium is the product of the concentration of the cell membrane permeable  substance and the cell volume, and the concentration of the cell membrane permeable substance is equal to the concentration of the cell membrane permeable substance in the storage solution. On the other hand, the cell volume at equilibrium, as described above, depends on the osmotic pressure of the cell membrane non- permeable substance in the storage solution ("A", "B" and "C" in Figure 2 correspond to the alphabets A, B and C in Figure 1, respectively) .

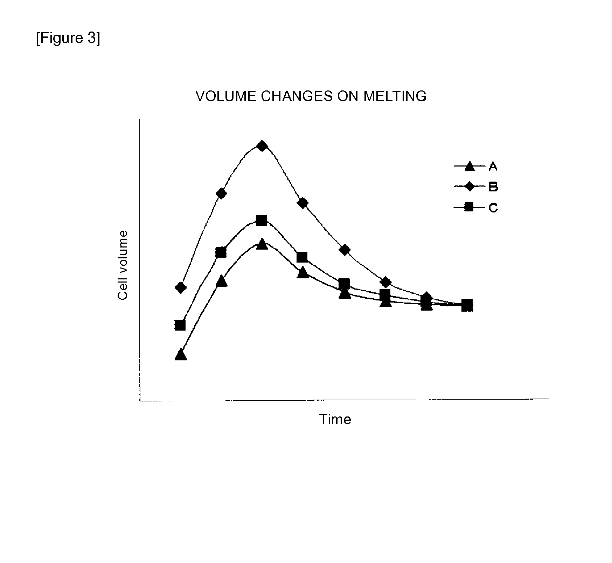

The change of the cell volume with the passage of time on melting the vitrified cells was believed to be shown in Figure 3. Melting is carried out by adding a heated diluent (which refers to a solution for dilution, and generally a culture medium for culture after melting is used) to the cells.

The vitrified intracellular fluid contains the cell membrane permeable substance, and the increased osmotic pressure is observed. Thus, the movement of water into the cells and the diffusion of the cell membrane permeable substance outward from the interior of the cells occur simultaneously at the initial stage of melting. The movement velocity of water is higher than the diffusion rate also at this point, and thus the cells expand temporally (often referred to hereinafter as "the water absorption phase"). Further, if water is absorbed into the cells over the limit volume until which the cells can expand, the cells are burst.

To what extent can the cells expand depends on the amount of the cell membrane permeable substance present in the cells, and the larger the amount, the larger the cells expand. It was regarded as described above that the amount of the cell membrane permeable substance present in the cells is determined by the vitrification time, the concentration of the cell membrane permeable substance in the vitrification medium and the osmotic pressure of the cell membrane non-permeable substance.

In this connection, A, B and C in Figure 3 show the volume change of the vitrified cells on melting at the timings corresponding to the respective alphabetical symbols in Figure 1. That is to say,

A: When cells are suspended in the vitrification medium and vitrified within 15 seconds, the volume change of the cells is little because of a small amount of the cell membrane permeable substance within the cells.

B: When cells are equilibrated in a hypotonic vitrification medium before vitrification, much amount of the cell membrane permeable substance is present within the cells due to the expansion of the cells, and the cell volume is expanded by water absorption. In this connection, if the cell volume is expanded over the limit volume, the cells are burst.

C: When cells are equilibrated in a hypertonic vitrification medium before vitrification, the amount of the cell membrane permeable substance within the cells is restrained to a low level due to the shrink of the cells, and the cells are not much expanded.

Thereafter, as the cell membrane permeable substance diffuses from the cells, the osmotic pressure within the cells is reduced and the volume of the cells is decreased (referred to hereinafter as "the diffusion phase") . When the concentrations of the cell membrane permeable substance become equivalent between the inside and the outside of the cells, the change of the cell volume stops (this step is often called "the equilibrium phase by the diluent", or simply "the equilibrium phase") .

In this connection, while it is thought that the difference of the osmotic pressure between the outside and inside of the cells is reduced and the expansion of the volume can be restrained by melting the cells in a hypertonic solution, this method requires the special preparation of a diluent and cannot necessarily be said convenient.

## DISTINCTION BETWEEN THE VITRIFICATION MEDIUM AND DAP213

DAP213 which has hitherto been generally used as the vitrification medium for primate ES/iPS cells was examined. The vitrification preservation of primate ES/iPS cells with DAP213 was herein examined according to the method disclosed in the home page of Riken CDB (Riken Center for Developmental Biology, Incorporated Administrative Agency) (Protocol for maintaining and culturing human pluripotent stem cell (2010)).

As is apparent from the protocol and the like, when DAP213 is used as a storage solution, it is necessary to complete the vitrification operation "within 15 seconds" after suspending the ES/iPS cells in the storage solution. At this time, the concentration of the cell permeable substance within the ES/iPS cells is thought not to have reached the equilibrium. It is known that if the operation time until vitrification exceeds 15 seconds, the survival rate of the ES/iPS cells is rapidly and prominently decreased and almost all the cells become extinct after 60 seconds.

While cryoprotective agents (that is, cell membrane permeable substances) contained in DAP213 comprises 2 M dimethyl sulfoxide, 1 M acetamide, and 3 M propylene glycol, none of them are ionized and thus contribution to osmotic pressure is 6000 mOsm.

DAP213 further comprises about 60% by volume of a culture medium, and if the osmotic pressure in the culture medium is estimated at 300 mOsm for convenience, contribution to the osmotic pressure of the whole DAP213 by a solute molecule in the culture medium becomes about 180 mOsm. Thus, the osmotic pressure of the whole DAP213 can be estimated in total at about 6180 mOsm.

Hereupon, it is predicated that the osmotic pressure which arises from a cell membrane non- permeable substance and influences the cell volume at equilibrium due to the storage solution is about 180 mOsm among about 6180 mOsm of the osmotic pressure of DAP213.

Thus, DAP213 was believed to behave as a hypotonic solution and expand cells at equilibrium.

In fact, it is observed in experiments with erythrocytes that in the suspensions of erythrocytes in saline solutions having various concentrations, the cell membrane is destroyed (hemolysis) when erythrocytes are suspended in a saline solution having a concentration of about 0.5% by weight or less. The saline solution having a concentration of about 0.5% by weight has an osmotic pressure of about 160 mOsm, which approximates to about 180 mOsm of DAP213.

It is thus thought that cells suspended in DAP213 and reached equilibrium after a fixed time may expand virtually to their limit volume notwithstanding some differences depending on cells. Then, a large amount of the protectant has been permeated into the cells. It was believed that the cells vitrified in this state and then melted will be burst in the water absorption phase, as described above.

It was judged from the investigation described above by the present inventor that in the preservation of the ES/iPS cells with DAP213 the phenomenon that the time required for suspending the cells in DAP213 and pouring the cells into liquid nitrogen seriously affects the survival rate of the cells may also arise from the other causes in addition to the strong cytotoxicity by a high concentration of the cryoprotective agent contained in the vitrification medium, which had hitherto been thought problematic.

Thus, the attention was first paid to the time required for reaching equilibrium of the ES/iPS cells.

It is predicated that while cells are generally left standing in a vitrification medium for 1 to 5 minutes for equilibration in ovules, fertilized eggs and embryos, the time required for equilibration is short in the ES/iPS cells due to their small volume.

Thus, it was supposed that the decrease of the survival rate in the preservation of the ES/iPS cells with DAP213 with the passage of time may be attributed to the approach of the cells to the equilibrium state and the increase of the cell membrane permeable substance within the cells before pouring the cells into liquid nitrogen.

That is to say, if the time after suspending the cells in the vitrification medium is within 15 seconds, the amount of the cell membrane permeable substance within the cells is little on vitrification, and the movement of water into the cells due to the osmotic pressure difference on melting and the change of the cell volume accompanying with the movement of water can be permitted. On the other hand, if the time after suspending the cells in the vitrification medium exceeds 15 seconds, the amount of the cell membrane permeable substance within the cells is increased with the passage of time and the cells to be burst by the intolerable volume change on melting is increased. Such hypothesis has been thought.

In order to validate the above described hypothesis, 3 DAP213s different in the concentration of the cell membrane non-permeable substance were prepared with PBS in place of a culture medium, and after mouse ES cells were suspended in the DAP213s and left standing for 90 seconds, the survival rates and the number of viable cells were confirmed by the trypan blue staining after dilution of the cells with the isotonic PBS without vitrification. The trypan blue staining is a method for judging the life or death of cells by utilizing the active excretion of a pigment from viable cells, and the cells negative to the staining are judged as viable cells and those positive to the staining as dead cells. However, the cells of which membrane structure was destroyed are counted neither as viable nor as dead cells and are reflected on a result as the decrease in the total cells. The survival rate was calculated from the ratio of the number of viable cells to the cell count before test as 100 (see Example 6 described below).

Consequently, the survival rate was reduced by 15% to 20% in the case (DAP-A) of replacing the basal medium of DAP213 with PBS so that the osmotic pressure becomes virtually equivalent to that of DAP213 (hypotonic) as compared with the case (DAP-B) which was prepared so that the final salt concentration is equivalent to the concentration in physiological environment (isotonic) and the case (DAP-C) which was prepared so that the final salt concentration is twice the concentration in physiological environment (hypertonic) in consideration of the dilution of the solution with the protectant. It was shown from this result that the survival rate of the cells in a hypotonic storage solution is lowered on dilution even if the cells are not vitrified. In addition, dead cells positive to trypan blue were scarcely observed. This observation indicated that the decrease in the viable cells is not attributed to the cytotoxicity of the cryoprotective agent, which was hitherto questioned, but due to osmotic pressure accompanied with the physical destruction of membrane structure. It was believed that the improvement of the survival rate by making the vitrification medium hypertonic is attributed to the control of the amount of the membrane permeable substance within the cells, which can withstand the influx of water into the cells on melting or dilution. It was shown that the preservation efficiency of the vitrification medium depends largely on the osmotic pressure and thus the survival rate is largely improved by making the storage solution hypertonic.

The present invention is based on such information.

## PROTECTION FROM CRYSTALLIZATION ACCOMPANIED WITH TEMPERATURE INCREASE

Furthermore, the present inventors have also examined the protection from crystallization accompanied with temperature increase in vitrification.

That is, the vitrification state is stable at a temperature of the glass transition temperature or less, but if the temperature is raised to the glass transition temperature or more, the cells are readily transferred from the glassy state to the crystalline state. This is believed that the molecule acquires kinetic energy and is spontaneously rearranged (crystallized) to a stable state, and the cells may be damaged by ice crystals generated at this time. Phase transition from the glassy state to the crystalline state causes the refraction and scattering of light, and the phenomenon called "devitrification" is observed, in which the appearance changes from transparent to opaque.

The glass transition temperature of the vitrification medium is generally lower than -80°C, and if cells, e.g. those which have been once vitrified, are preserved at -80°C, the cells are devitrified and damaged by ice crystals to lead to the death of almost all the cells.

Devitrification phenomenon is observed on melting the vitrified cells, and it is thus believed that the cells are damaged by ice crystals.

The present inventors have also found that the ice crystals can be suppressed from growing largely at crystal transition and the damage to the cells can be reduced by adding ice crystal growth inhibitors such as sericin and antifreeze proteins. The present invention is also based on such information.

## VITRIFICATION MEDIUM FOR CELLS

As described above, the vitrification medium for cells according to the present invention is the one comprising a cell membrane permeable substance and a cell membrane non-permeable substance, in which

the content of the cell membrane permeable substance is in the range of 30 to 50% by volume, and

the osmotic pressure generated in association with the cell membrane non-permeable substance, which is a fraction of the total osmotic pressure on the cell membrane on suspending the cells in the vitrification medium, is in the range of 280 mOsm or more. In this connection, the vitrification medium for cells can be said, in other words, a composition for vitrification preservation for cells, particularly a liquid composition for vitrification preservation for cells.

Here, the cells to be preserved are not particularly limited provided the cells can be preserved with the vitrification medium according to the present invention and include, for example, cell lines, primary cell culture, normal cells, germ cells, ES cells and iPS cells. Furthermore, biological tissues such as pancreatic islets, which have such a size as can be treated equally to cells, can be regarded as the target for preservation. In addition, the biological species include microorganisms, plants and animals, and the animal species include particularly mammals, birds, fish and insects. Mammals include particularly rodents such as mice, and primates such as simians and humans. Above all, ES/iPS cells which were difficult of efficient preservation by the slow freezing method or the vitrification method with DAP213 are preferred, and primate ES/iPS cells (primate ES cells or primate iPS cells) are more preferred.

CELL MEMBRANE PERMEABLE SUBSTANCE

In the present invention, the cell membrane permeable substance is used as a cryoprotective agent for the vitrification of a storage solution and an intracellular fluid. The cell membrane permeable substance includes, for example, diols such as ethylene glycol (EG), propylene glycol (PG), 1,3-propanediol (1,3-PD), butylene glycol (BG), isoprene glycol (IPG) and dipropylene glycol (DPG), triols such as glycerin, and dimethyl sulfoxide (DMSO), and is not limited to those described above, provided that the substance is the one which permeates the cell membrane to accomplish the object described above and has low toxicity to cells and organisms. The cell membrane permeable substance is one or more selected from the group consisting of ethylene glycol (EG), propylene glycol (PG), 1,3-propanediol (1,3-PD), butylene glycol (BG), isoprene glycol (IPG), dipropylene glycol (DPG), glycerin and dimethyl sulfoxide (DMSO).

Among them, it is preferred to use, as the cell membrane permeable substance, EG, PG, glycerin and DMSO for the reasons such as the glass forming ability, the permeability to the cell membrane, the protective effect to the cells and the utility results to storage solutions, more preferably EG, PG and DMSO, and further preferably EG and PG. These can be used in combination of 2 or more. The combination is preferably of DMSO and PG, DMSO and EG, and EG and PG. The substance in individual use is preferably EG.

The content of the cell membrane permeable substance is typically in the range of 30 to 50% by volume, preferably in the range of 33 to 47% by volume, and more preferably in the range of 35 to 45% by volume. Here, if the content described above is less than 30% by volume, the storage solution forms ice crystals without vitrification, which may damage the cells. On the other hand, if the above described content exceeds 50% by volume, the cell structure may be destroyed due to excessive dehydration before vitrification, or the survival rate of the cells may be lowered due to the stagnation of physiological reaction which requires water as an essential component. In addition, excessive osmotic pressure may generate on melting, which causes the destruction of the cell membrane.

When DMSO and the other protectant such as PG are used in combination as the cell membrane permeable substance, the volume ratio is preferably in the range of 1 : 5 to 2 : 1, and more preferably 1 : 3 to 1 : 1. Also, when DMSO and EG are used in combination, the volume ratio is preferably in the range of 1 : 5 to 2 : 1, and more preferably 1 : 3 to 1 : 1. Furthermore, when EG and PG are used in combination, the volume ratio is preferably in the range of 1 : 100 to 100 : 1, and more preferably 1 : 10 to 10 : 1.

Contribution to osmotic pressure of the cell membrane permeable substance, of which content is 30 to 50% by volume, largely varies depending on the molecular weights of the cell membrane permeable substance and cannot be indiscrimately defined. However, when using EG alone, it is generally in the range of 5000 to 9000 mOsm, and when using PG alone, it is generally in the range of 4000 to 7000 mOsm. Further, the contribution to osmotic pressure on using DMSO and PG in a volume ratio of 1 : 5 to 2 : 1 is generally in the range of 4000 to 7000 mOsm, the contribution to osmotic pressure on using DMSO and EG in a volume ratio of 1 : 5 to 2 : 1 is generally in the range of 4000 to 9000 mOsm, and the contribution to osmotic pressure on using EG and PG in a volume ratio of 1 : 100 to 100 : 1 is generally in the range of 4000 to 9000 mOsm.

CELL MEMBRANE NON-PERMEABLE SUBSTANCE

In the present invention, the cell non-membrane permeable substance is used for the purpose of adjusting the osmotic pressure to physiological environment and promoting vitrification in addition to controlling the osmotic pressure. Thus, the cell membrane non-permeable substance may comprise a substance for adjusting osmotic pressure to physiological environment (physiological environment adjusting substance) and a substance for promoting vitrification (vitrification promoting substance).

The substance for adjusting osmotic pressure to physiological environment may include, for example, salts such as sodium salts, potassium salts and phosphate salts, saccharides such as glucose and sucrose, proteins such as albumin, minerals such as magnesium and calcium, and the like. Above all, sodium chloride, potassium chloride, disodium hydrogen phosphate and potassium dihydrogen phosphate are preferably used.

The substance for promoting vitrification may include, for example, whole saccharides including monosaccharides such as glucose, galactose and fructose; disaccharides such as trehalose, sucrose and maltose; trisaccharides such as raffinose; polysaccharides such as cellulose and starch; sugar alcohols such as xylitol, sorbitol, erythritol and mannitol; and hydrophilic polymers such as polyethylene glycol, polyvinyl alcohol, polyvinylpyrrolidone and Ficolls. Preferably, glucose, sucrose, maltose, trehalose and polyethylene glycol are used, and more preferably, glucose, sucrose, maltose, trehalose, polyethylene glycol are used. Among them, trehalose and sucrose are more preferred, and trehalose is particularly preferred.

According to one preferred embodiment of the present invention, the cell membrane non-permeable substance is one or more selected from the group consisting of sodium chloride, potassium chloride, disodium hydrogen phosphate, potassium dihydrogen phosphate, monosaccharides, disaccharides, trisaccharides, polysaccharides, sugar alcohols, polyethylene glycol, polyvinyl alcohol and polyvinylpyrrolidone. More preferably, the cell membrane non-permeable substance comprises a physiological environment adjusting substance selected from the group consisting of sodium chloride, potassium chloride, disodium hydrogen phosphate and potassium dihydrogen phosphate and a vitrification promoting substance selected from the group consisting of trehalose and sucrose.

When, among the cell membrane non- permeable substances, the substances for adjusting osmotic pressure to physiological environment, particularly, sodium chloride, potassium chloride, disodium hydrogen phosphate or potassium dihydrogen phosphate are not contained, the survival rate of cells may be lowered. On the other hand, the substance for promoting vitrification, which is not the essential ingredient, is preferred to be added to the vitrification medium, since the vitrification ability of the vitrification medium may be lowered by the remaining culture medium or the like resulting in the formation of ice crystals and thus damaging the cells. Furthermore, the risk of the phase transition from the glassy state to the crystalline state due to temporary ascent of temperature during preservation can be reduced by raising the glass transition temperature (Tg) of the vitrification medium.

The content of the cell membrane non-permeable substance is required to be the one in which osmotic pressure induced by the cell membrane non-permeable substance among the total osmotic pressure generating on suspending the cells in the vitrification medium is 280 mOsm or more. If the osmotic pressure is less than 280 mOsm, the cells suspended in the storage solution are expanded along with the approach to equilibrium and the cells are likely to be burst on the movement of water into the cells on melting. Thus, when the osmotic pressure is less than 280 mOsm, in order to get high preservation efficiency, the vitrification must be completed before equilibration of the cells with the storage solution and the preservation efficiency will vary depending on the skills of operators.

The osmotic pressure arising from the cell membrane non-permeable substance is desirable that the vitrification preservation agent contains the cell membrane non-permeable substance so that the osmotic pressure is preferably in the range of 600 mOsm or more, more preferably 800 mOsm or more. The osmotic pressure is generally sufficient to be in the range of 1500 mOsm or less, but it is generally desirable to be in the range of 1000 mOsm or less by reason of the harmfulness of salts in high concentrations to the cells or of the solubility limit of saccharides or in order to avoid lowering the stability of the solution due to the lowering of temperature, particularly to avoid deposition of lysate.

Hence, in order to accomplish the osmotic pressure described above, the vitrification medium according to the present invention preferably comprises, the cell membrane non- permeable substance, e.g., trehalose having a concentration of 0.2 M to 1 M in the storage solution.

The specific example of the preferred combination contains a cell membrane permeable substance in the range of 30 to 50% by weight, preferably 40% by weight, ten times concentrated phosphate buffered saline (10×PBS) as a physiological environment adjusting substance, which is a cell membrane non-permeable substance, in the range of 5 to 20% by volume or an amount that the osmotic pressure attributed to 10×PBS is in the range of 140 to 600 mOsm, and further a vitrification promoting substance, which is a cell membrane non-permeable substance, in the range of 0 to 1.5 M, preferably 0.2 to 1 M or an amount that the osmotic pressure attributed to the vitrification promoting substance is in the range of 0 to 1500 mOsm, preferably 200 to 1000 mOsm. Hereby, the osmotic pressure induced by the cell membrane non-permeable substance can be adjusted to 280 mOsm or more, preferably 600 mOsm or more, more preferably 800 mOsm or more.

At this time, there can be used as the cell membrane permeable substance, ethylene glycol (EG), propylene glycol (PG), 1,3-propanediol (1,3-PD), butylene glycol (BG), isoprene glycol (IPG), dipropylene glycol (DPG), glycerin and dimethyl sulfoxide (DMSO) independently or in combination, and preferably EG or PG alone or a combination of DMSO and PG in the ratio of 1 : 3 to 1 : 1, of DMSO and EG in the ratio of 1 : 3 to 1 : 1, or of EG and PG in the ratio of 1 : 10 to 10 : 1 is used.

The specific example of the composition of the ten times concentrated phosphate buffered saline used as the physiological environment adjusting substance includes a solution comprising 1370 mM sodium chloride, 27 mM potassium chloride, 100 mM disodium hydrogen phosphate and 17.6 mM potassium dihydrogen phosphate, and the plural compositions further comprising magnesium chloride and calcium chloride in addition to the composition described above are known, either of which may be used. The ten times concentrated phosphate buffered saline is preferably used in the present invention.

ICE CRYSTALS GROWTH INHIBITOR

There can be used, for example, a group of proteins which is known as an antifreeze protein (Antifreeze protein, AFP) as the ice crystal growth inhibitor. AFP suppresses the growth of crystals by adsorbing on the crystal plane or attracting water molecule to the surrounds of it. AFP includes the ones, for example, derived from animals, plants and insects, and the commercial products can be appropriately used. However, AFP is difficult to obtain it in a large amount at one time and generally expensive. Thus, a substitute of AFP such as sericin can be used as the ice crystal growth inhibitor.

Above all, sericin has hitherto been known to protect cells and proteins from freezing stress and has now been found to suppress the growth of ice crystals. That is, the present inventors have now found unexpectedly that sericin added to the vitrification medium for cells has an effect to suppress the growth of ice crystals.

Thus, according to one preferred embodiment of the present invention, the vitrification medium for cells according to the present invention further comprises an ice crystal growth inhibitor selected from the group consisting of an antifreeze protein and sericin. More preferably, the ice crystal growth inhibitor is sericin.

Sericin used in the present invention may be the one derived from natural products or synthesized by the usual chemical and/or genetic engineering techniques. Sericin may also be the hydrolyzate of sericin with acids, alkalis or enzymes.

Sericin derived from natural products can be extracted from cocoons or raw silk by the usual extraction methods. Specifically, sericin may be obtained from a raw material such as cocoons, raw silk or silk fabrics by partial hydrolysis with hot water, acids, alkalis or enzymes followed by extraction, and products further purified to a higher degree are preferred for the purpose of obtaining the one having a definite quality and a stable culture and preservation state. Furthermore, sericin may be a solid such as powder or granule or a liquid having sericin melted or suspended in water or a buffer solution.

In the present invention, the average molecular weight of sericin is not specifically limited, but is preferably in the range of 5,000 to 100,000 and more preferably 10,000 to 50,000.

The amount of sericin to be added is preferably in the range of 0.1 to 10%, more preferably 1 to 5%, particularly preferably 3 to 5% on the basis of the weight to the total amount of the vitrification medium.

OTHER INGREDIENTS

The vitrification medium for cells according to the present invention comprises the ingredients described above, but desirably contains no animal-derived ingredients, because the animal-derived ingredients such as serum may cause viral contamination.

Furthermore, the vitrification medium for cells according to the present invention may comprise the other ingredients which are within the range of not damaging the effects of the present invention. The other ingredients include, for example, amino acids, hormones, cytokines, antioxidants, pH buffers and pH regulating agents.

That is, the vitrification medium of the present invention can be applied in place of the vitrification medium described in plural well known protocols used on the vitrification preservation of cells such as Riken CDB protocol (e.g., Protocol of culture practice of human pluripotent stem cells 2010 (available from http://www.cdb.riken.go.jp/)).

VITRIFICATION METHOD

According to the present invention, as described above, there is provided a vitrification method for cells comprising suspending cells in the vitrification medium for cells of the present invention before rapidly cooling the vitrification medium containing the cells with liquid nitrogen for vitrification, in which
the osmotic pressure generated in association with the cell membrane non-permeable substance, which is a fraction of the total osmotic pressure on the cell membrane on suspending the cells in the vitrification medium, is in the range of 280 mOsm or more.

By way of a specific example of the method according to the present invention, cells as the target for preservation are recovered, centrifuged for removing the culture medium and then suspended in the vitrification medium according to the present invention, a small portion of the suspension was charged into a sealed tube, which is rapidly cooled with liquid nitrogen or the like for vitrification. Preservation is carried out in the vapor phase or the liquid phase at the temperature of liquid nitrogen (-190 to -196°C).

Melting is carried out by directly adding the heated dilution to the sealed tube, then further dilution and washing with a culture medium were carried out, and the dilution was removed by centrifugation before culture of the suspension in a culture medium.

A culture medium can be used as the diluent. In this connection, it is preferred to use a dilution having an increased osmotic pressure when the concentration of the cell membrane non-permeable substance in the storage solution cannot be sufficiently increased or the cell will be burst by the osmotic pressure difference due to the fragileness of cell membrane or the like. At this moment, the solute for adjusting osmotic pressure may be a cell membrane permeable substance or a cell membrane non-permeable substance. Generally, saccharides, particularly glucose and sucrose are used as the solute, and any one which will not be harmful to cells and can adjust osmotic pressure may be used.

EXAMPLES

The present invention is described in further detail with reference to the following examples, but the present invention is not limited thereto. Unless otherwise specified, % representing a composition is regarded as % by volume.

EXAMPLE 1:

Materials:

Cell membrane permeable substance:

· Dimethyl sulfoxide (DMSO)
· Ethylene glycol (EG)
· Propylene glycol (PG)
· Butylene glycol (BG)
· Isoprene glycol (IPG)
· Diprepylene glycol (DPG)
· Polyethylene glycol #400 (PEG)

Used cells:

· Mouse ES cell line strain D3 (available from ATCC)
· Common marmoset ES cell line, strain CMES40 (available from Riken Cell Bank)
* Common marmoset ES cell line, which was the cell established by Sasaki in Central Institute for Experimental Animals, was available from Riken Cell Bank and used.
· Feeder cell line, strain STO (available from Riken Cell Bank)

Composition of Culture Mediums used:

1) Mouse ES cell maintenance culture medium

· KnockOut DMEM (manufactured by GIBCO)
· 20% Knockout Serum Replacement (manufactured by GIBCO)
· 0.1 mM Non-essential amino acids (manufactured by GIBCO)
· 0.1 mM 2-mercaptoethanol (manufactured by GIBCO)
· 2 mM L-glutamine (manufactured by GIBCO)
· 1000 U/ml LIF (manufactured by Wako Pure Chemical Industries, Ltd.)

2) Common marmoset ES cell maintenance culture medium

· KnockOut DMEM (manufactured by GIBCO)
· 20% Knockout Serum Replacement (manufactured by GIBCO)
· 0.1 mM Non-essential amino acids(manufactured by GIBCO)
· 0.1 mM 2-mercaptoethanol (manufactured by GIBCO)
· 2 mM L-glutamine (manufactured by GIBCO)
· 4 ng/ml bFGF (manufactured by Wako Pure Chemical Industries, Ltd.)

Control Section of Vitrification Medium (DAP213):

· After acetamide (0.59 g) was dissolved in KnockOut DMEM (6 ml), the solution was sterilized by filtration;
· DMSO (1.42 ml) and PG (2.2 ml) were added;
· Dilution with Knockout DMEM to a volume of 10 ml.

<u>Test Section of Vitrification Medium:</u>

|  | |
|---|---|
| · Milli-Q | 400 to 800 μl |
| · 10×PBS | 100 μl |
| · cell membrane permeable substance | 100 to 500 μl |
| Total | 1000 μl |

<u>(1-1) Examination of Vitrification Ability of Cell Membrane Permeable Substance</u>

Kinds of cell membrane permeable substances greatly influencing the toxicity of the vitrification medium were examined. While many vitrification promoting substances are known, it is  preferred that the substances have no toxicity or mutagenicity and permeability into cells. The present inventors have focused from the standpoint on diols having low molecular weights which have been used as the raw materials of cosmetics. Several diols such as EG and PG have already been used as a cryoprotective agent and has been confirmed to have the safety to skin, so that the cytotoxicity of these diols is expected to be in low levels. Thus, the capabilities of the six diols including EG, PG, BG, IPG, DPG and PEG in addition to DMSO as the vitrification medium were examined.

The 10 to 50% solutions of 6 diols (EG, PG, BG, IPG, DPG and PEG) and of DMSO were prepared and confirmed to be vitrified by rapid cooling with liquid nitrogen. Specifically, respective cell membrane permeable substances are dissolved in PBS to concentrations of 10, 20, 30, 40 and 50%, and the solution was stained with a small amount of phenol red in order to readily judge the vitrification. The solution thus prepared was divided into 200 μl portions in cryo-tubes, which were dipped into liquid nitrogen for rapid cooling. The tubes were dipped into liquid nitrogen for 5 minutes or more to stabilize the temperature before observation of each tube.

The results were as follows.

PBS as the negative control was not vitrified and frozen as a polycrystalline substance. The polycrystalline substance formed from PBS was confirmed by light scattering on the crystal interface and opaque appearance (figures were not shown). On the other hand, DAP213 as the positive control generally shows transparency, from which the glassy state of DAP213 could be confirmed. Furthermore, phase transition to an opaque crystalline solid was observed by temperature increase.

It was shown that vitrification was brought about by adding any one of the diols and DMSO tested in a concentration of 40% or more. Particularly, PG, IPG, BG and DPG were confirmed to be vitrified even in the 30% solution.

<u>(1-2) Influence of Cell Membrane Permeable Substance on Cells</u>

<u>(1-2-1) Examination of Cytotoxicity and Influence on Differentiation with Common Marmoset (CM) ES cells</u>

In order to examine the influence of the cell membrane permeable substance on CMES cells, culture was conducted by adding the cell membrane permeable substances, respectively, in a concentration of 2% in a culture medium for CMES and the condition of the colony was observed.

Specifically, the experiment was conducted as follows:

· CMES cells were cultured with reference to "Establishment of novel embryonic stem cell lines derived from the common marmoset" (Sasaki E, Hanazawa K, Kurita R, Akatsuka A, Yoshizaki T, Ishii H, Tanioka Y, Ohnishi Y, Suemizu H, Sugawara A, Tamaoki N, Izawa K, Nakazaki Y, Hamada H, Suemori H, Asano S, Nakatsuji N, Okano H, Tani K. Stem Cells. 2005 Oct; 23 (9) : 1304- 13) .

· CMES cells were seeded in a φ35 mm dish to which feeder cells had preliminarily been adhered, and the cell membrane permeable substance was added in an amount corresponding to 2% of the amount of the culture medium.

· After 3 days, microscopic observation was conducted, and the presence of the growth suppression and differentiation promotion on CMES cells was examined.

As a result, the nature of the colony morphology was good in the control (no addition) and DAP213, DMSO, EG, PG and PEG (figures were not shown).

(1-2-2) Measurement of Vitrification Preservation Efficiency with Mouse ES Cells

Among the seven cell membrane permeable substances, the four substances (DMSO, EG, PG, PEG) which showed no differentiation promoting effect on the CMES cells were used alone or in combination of 1 : 1 so that the total amount of the cell membrane permeable substance is 40%, and the vitrification protecting effects of the respective cell membrane permeable substances were confirmed. Since the cell count of the CMES cells was difficult, mouse ES cells were used in the test.
The mouse ES cells after vitrification and melting were seeded, and the state of the cells after culture was confirmed by microscopic observation.

Specifically, the experiment was conducted as follows:

· Mouse ES cells were cultured according to the ordinary method.
· The mouse ES cells recovered were counted, divided into $10^6$ cell portions in 1.5 ml tubes, and centrifuged at 1500 rpm for 5 minutes.
· The supernatant was removed, and the cells were suspended in 200 $\mu$l of the vitrification medium.
· After suspension, DAP213 and the test fluid were dipped into liquid nitrogen after 15 seconds and 60 seconds, respectively, for vitrification.
· The tubes were left standing for 5 minutes or more to stabilize the temperature.
· The tubes were taken out liquid nitrogen, and the cells were diluted with 1800 $\mu$l of the culture medium preliminarily heated to 37°C and rapidly suspended for melting.
· A portion of the suspension was sampled for counting the cells.
· On counting the cells, the life or death of cells was judged from the index of the selective excretive ability of the viable cells to trypan blue and the survival rate was calculated.
· The remaining cells were seeded in a 24 well plate, and the state after 3 days was confirmed by microscopic observation.

When the cell membrane permeable substance was used alone, the biggest colony was observed in EG, and secondly in PG. The least number of colonies were observed in DMSO, but all the cells died and no colonies were observed in PEG. When the cell membrane permeable substance was used in combination, many colonies were observed in DMSO+EG, DMSO+PG, DMSO+PEG and EG+PEG (figures were not shown).
As to the five cell membrane permeable substances of EG, DMSO+EG, DMSO+PG, DMSO+PEG and EG+PEG, in the morphological observation of which many colonies were found, the survival rate after vitrification was measured. The 40% solution of each cell membrane permeable substance in PBS was prepared, and the mouse ES cells was suspended in the solution, left standing for 60 seconds, and dipped into liquid nitrogen for vitrification.

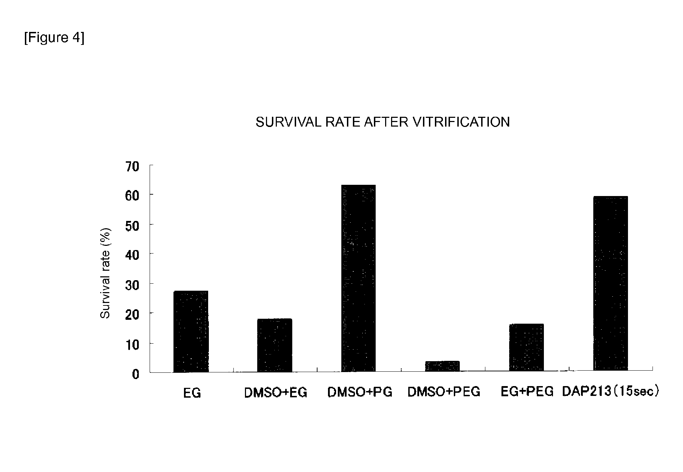

When the survival rate after melting was measured, it was particularly high in DMSO+PG (Figure 4).
While the EG alone is most excellent among the cell membrane permeable substances employing no DMSO, the survival rate was substantially lowered as compared with DMSO+PG.

EXAMPLE 2:

Materials:

Cell membrane permeable substance:

· Dimethyl sulfoxide (DMSO)
· Propylene glycol (PG)

Used cells:

· Mouse ES cell line D3 strain (available from ATCC)

Composition of Culture Mediums used:

Mouse ES cell maintenance culture medium:

· KnockOut DMEM (manufactured by GIBCO)
· 20% Knockout Serum Replacement (manufactured by GIBCO)
· 0.1 mM Non-essential amino acids (manufactured by GIBCO)
· 0.1 mM 2-mercaptoethanol (manufactured by GIBCO)
· 2 mM L-glutamine (manufactured by GIBCO)
· 1000 U/ml LIF (manufactured by Wako Pure Chemical Industries, Ltd.)

Vitrification medium:

| | |
|---|---|
| · Milli-Q | 500 $\mu$l |
| · 10×PBS | 100 $\mu$l |
| · DMSO | 80 to 200 $\mu$l |
| · PG | 200 to 320 $\mu$l |
| Total | 1000 $\mu$l |

Test fluid:

The contents of DMSO and PG were prepared so that the total concentration is 40%.
[Table 1]

| Concentration of DMSO | Added Amount of DMSO | Added Amount of PG |
|---|---|---|
| 8% | 80 | 320 |
| 10% | 100 | 300 |
| 12% | 120 | 280 |
| 14% | 140 | 260 |
| 20% | 200 | 200 |

DAP213:

· After acetamide (0.59 g) was dissolved in KnockOut DMEM (6 ml), the solution was sterilized by filtration;
· DMSO (1.42 ml) and PG (2.2 ml) were added;
· Dilution with Knockout DMEM to a volume of 10 ml.

(2-1) Vitrification of Mouse ES Cells

Mouse ES cells were vitrified with a storage solution, in which the addition ratio of DMSO and PG was changed, and the survival rates after melting were compared.
In the test section, the time from suspending the cells to vitrification was set 60 seconds, and in the control section the cells were vitrified within 15 seconds after suspension with DAP213.
Specifically, experiment was conducted as follows:

· Mouse ES cells were cultured according to the ordinary method.
· The mouse ES cells recovered were counted, divided into $10^6$ cell portions in 1.5 ml tubes, and centrifuged at 1500 rpm for 5 minutes.
· The supernatant was removed, and the cells were suspended in 200 $\mu$l of the vitrification medium.
· After suspension, DAP213 and the test fluid were dipped into liquid nitrogen after 15 seconds and 60 seconds, respectively, for vitrification.
· The tubes were left standing for 5 minutes or more to stabilize the temperature.

· The tubes were taken out from liquid nitrogen, and the cells were diluted with 1800 µl of the culture medium preliminarily heated to 37°C and rapidly suspended for melting.

· A portion of the suspension was sampled for counting the cells.

· On counting the cells, the life or death of cells was judged from the index of the selective excretive ability of the viable cells to trypan blue and the survival rate was calculated.

· The remaining cells were seeded in a 24 well plate, and the state after 3 days was confirmed by microscopic observation.

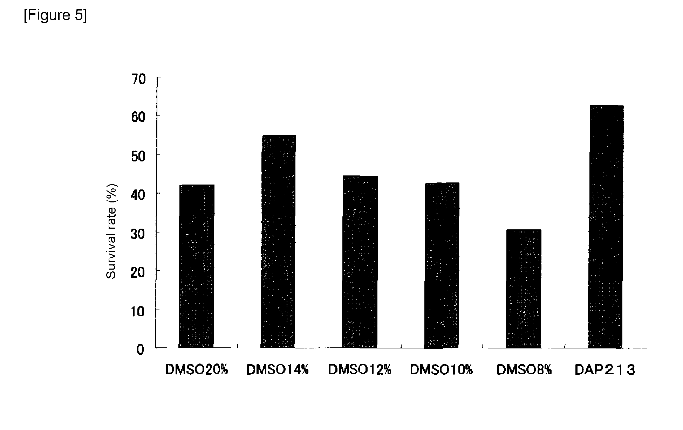

The results were as shown in Fig. 5.

The survival rate was not influenced even if the content of DMSO was reduced to 10%, but the decrease of the survival rate was observed if the content of DMSO was reduced to 8%. Thus, it was revealed that the content of DMSO can be reduced more than that of the current product DAP213 (14.2%).

EXAMPLE 3:

Materials:

Cell Membrane Permeable Substance:

· Dimethyl sulfoxide (DMSO)
· Propylene glycol (PG)
· Acetamide

Used cells:

· Mouse ES cell line D3 strain (available from ATCC)

Composition of Culture Mediums used:

Mouse ES cell maintenance culture medium:

· KnockOut DMEM (manufactured by GIBCO)
· 20% Knockout Serum Replacement (manufactured by GIBCO)
· 0.1 mM Non-essential amino acids (manufactured by GIBCO)
· 0.1 mM 2-mercaptoethanol (manufactured by GIBCO)
· 2 mM L-glutamine (manufactured by GIBCO)
· 1000 U/ml LIF (manufactured by Wako Pure Chemical Industries, Ltd.)

DAP213:

· After acetamide (0.59 g) was dissolved in KnockOut DMEM (6 ml), the solution was sterilized by filtration;
· DMSO (1.42 ml) and PG (2.2 ml) were added;
· Dilution with Knockout DMEM to a volume of 10 ml.

DP23:

· Dilution of Knockout DMEM with 1.42 ml of DMSO and 2.2 ml of PG to a volume of 10 ml.

(3-1) Examination of Efficacy of Acetamide

The survival rates of the mouse ES cells vitrified within 15 seconds and after 60 seconds from suspension with DAP213 containing acetamide and the same one but containing no acetamide (DP23) were measured.

Specifically, experiment was conducted as follows:

· Mouse ES cells were cultured according to the ordinary method.
· The mouse ES cells recovered were counted, divided into $10^6$ cell portions in 1.5 ml tubes, and centrifuged at 1500 rpm for 5 minutes.
· The supernatant was removed, and the cells were suspended in 200 µl of the vitrification medium.

· After suspension, DAP213 and the test fluid were dipped into liquid nitrogen after 15 seconds and 60 seconds, respectively, for vitrification.
· The tubes were left standing for 5 minutes or more to stabilize the temperature.
· The tubes were taken out from liquid nitrogen, and the cells were diluted with 1800 µl of the culture medium preliminarily heated to 37°C and rapidly suspended for melting.
· A portion of the suspension was sampled for counting the cells.
· On counting the cells, the life or death of cells was judged from the index of the selective excretive ability of the viable cells to trypan blue and the survival rate was calculated.

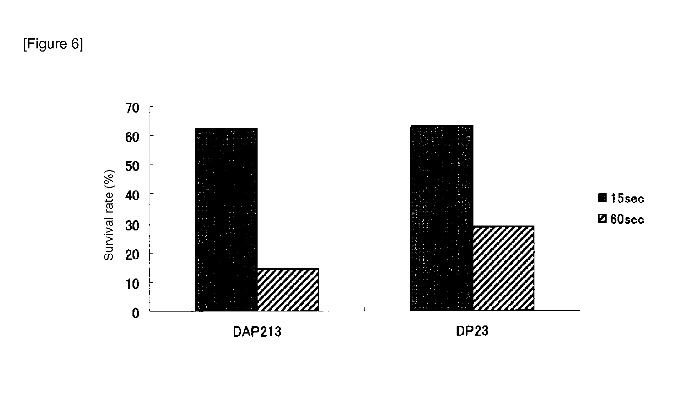

The results were as shown in Fig. 6.
The improvement of the survival rate due to acetamide was not recognized in the case of vitrification within 15 seconds, and the survival rate was reduced by acetamide in the case of vitrification after 60 seconds.

EXAMPLE 4:

Materials:

Vitrification Promoting Substance:

· D- (+)- Glucose
· Sucrose
· D- (+)- Maltose- monohydrate
· D- (- )- Fructose
· D- (+)- Trehalose- dihydrate
· D- (+)- Raffinose- pentahydrate
· Erythritol
· Xylitol
· D-Sorbitol
· D- (- )- Mannitol

Cell Membrane Permeable Substance:

· Dimethyl sulfoxide (DMSO)

Vitrification Medium:

| | |
|---|---|
| · Solution of Vitrification Promoting Substance | 300 to 500 µl |
| · DMSO | 300 µl |
| · PBS | 200 to 400 µl |
| Total | 1000 µl |

(4-1) Examination of Vitrification Promoting Substance

The efficiencies of 10 saccharides and sugar alcohols having low toxicity and excellent glass forming ability were examined by comparing the efficiencies of the vitrification promoting substances.

Specifically, the experiment was conducted as follows:

· As the vitrification promoting substance, the following saccharides were examined: glucose, sucrose, maltose, fructose, trehalose, raffinose, erythritol, xylitol, sorbitol and mannitol.
· The vitrification promoting substance was dissolved in 30% DMSO/PBS to a concentration of 0.3 M and 0.6 M respectively.
· The solution thus prepared was divided into 200 μl portions in cryo-tubes, which were dipped into liquid nitrogen for rapid cooling. The tubes were dipped into liquid nitrogen for 5 minutes or more to stabilize the temperature before observation of each tube.

When a variety of saccharides/sugar alcohols were added to the DMSO solution of the concentration (30%) in which no vitrification occurs and rapidly cooled in liquid nitrogen, it was confirmed that the solution is vitrified by the addition of 0.3 M trehalose or raffinose (figures were not shown). However, since raffinose has low solubility at low temperature and is liable to produce deposits, it was believed that trehalose is most excellent as the vitrification promoting substance.

EXAMPLE 5:

Materials:

Cell Membrane Non-Permeable Substance:

· Dimethyl sulfoxide (DMSO)
· Propylene glycol (PG)

Vitrification Promoting Substance:

· D- (+)- trehalose- dihydrate

Used cells:

· Mouse ES cell line D3 strain (available from ATCC)

Composition of Culture Mediums used:

Mouse ES cell maintenance culture medium:

· KnockOut DMEM (manufactured by GIBCO)
· 20% Knockout Serum Replacement (manufactured by GIBCO)
· 0.1 mM Non-essential amino acids (manufactured by GIBCO)
· 0.1 mM 2-mercaptoethanol (manufactured by GIBCO)
· 2 mM L-glutamine (manufactured by GIBCO)
· 1000 U/ml LIF (manufactured by Wako Pure Chemical Industries, Ltd.)

Vitrification Medium:

It was prepared according to the following table.
[Table 2]

| | DMSO | PG | Trehalose (*) | Milli-Q | 10xPBS | Total |
|---|---|---|---|---|---|---|
| 1) Physiological salt (isotonic) | 100 | 100 | 0 | 250 | 50 | 500 |
| 2) High concentration salt (hypertonic) | 100 | 100 | 0 | 200 | 100 | 500 |
| 3) Physiological salt + trehalose (hypertonic) | 100 | 100 | 150 | 100 | 50 | 500 |
| 4) No salt + trehalose (hypertonic) | 100 | 100 | 300 | 0 | 0 | 500 |
| (*) Trehalose is dissolved in Milli-Q so that the concentration is 1 M. | | | | | | |

(5-1) Addition Effect of Trehalose

Mouse ES cells were suspended in an experimentally produced storage solution, left standing for 60 seconds before vitrification, and the survival rate after melting was measured.

Specifically, experiment was conducted as follows:

· Mouse ES cells were cultured according to the ordinary method.
· The mouse ES cells recovered were counted, divided into $10^6$ cell portions in 1.5 ml tubes, and centrifuged at 1500 rpm for 5 minutes.
· The supernatant was removed, and the cells were suspended in 200 µl of the vitrification medium.
· After suspension, DAP213 and the test fluid were dipped into liquid nitrogen after 15 seconds and 60 seconds, respectively, for vitrification.
· The tubes were left standing for 5 minutes or more to stabilize the temperature.
· The tubes were taken out from liquid nitrogen, and the cells were diluted with 1800 µl of the culture medium preliminarily heated to 37°C and rapidly suspended for melting.
· A portion of the suspension was sampled for counting the cells.
· On counting the cells, the life or death of cells was judged from the index of the selective excretive ability of the viable cells to trypan blue and the survival rate was calculated.

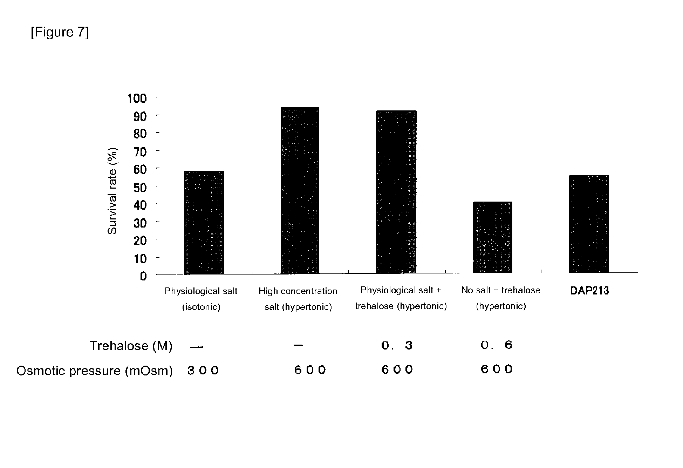

The results were as shown in Fig. 7.
The cells vitrified in a storage solution having trehalose added thereto exhibited the survival rate of about 90%. However, the mouse ES cells of which osmotic pressure was controlled not with trehalose but with the salt (PBS) also exhibited the high survival rate, and it was revealed that osmotic pressure greatly influences the preservation efficiency of the vitrification medium. But when osmotic pressure was prepared with a saccharide in the absence of a salt, the survival rate of the mouse ES cells was not improved, and it was suggested that the environment for maintaining the ion balance within cells such as sodium ion.

EXAMPLE 6:

Materials:

Cell Membrane Non-Permeable Substance:

· Dimethyl sulfoxide (DMSO)
· Propylene glycol (PG)
· Acetamide

Used Cells:

· Mouse ES cell line D3 strain (available from ATCC)

Composition of Culture Mediums used:

Mouse ES cell maintenance culture medium:

· KnockOut DMEM (manufactured by GIBCO)
· 20% Knockout Serum Replacement (manufactured by GIBCO)
· 0.1 mM Non-essential amino acids (manufactured by GIBCO)
· 0.1 mM 2-mercaptoethanol (manufactured by GIBCO)
· 2 mM L-glutamine (manufactured by GIBCO)
· 1000 U/ml LIF (manufactured by Wako Pure Chemical Industries, Ltd.)

Vitrification Medium:

· DAP213:

[Table 3]

| | DMSO | PG | Acetamide (*) | 10xPBS | Milli-Q | Total |
|---|---|---|---|---|---|---|
| DAP-A | 710 | 1100 | 590 | 320 | 2280 | 5000 |
| DAP-B | 710 | 1100 | 590 | 500 | 2100 | 5000 |
| DAP-C | 710 | 1100 | 590 | 1000 | 1600 | 5000 |
| (*) Acetamide is dissolved in Milli-Q so that the concentration is 0.5 g/ml. | | | | | | |

(6-1) Influence of Osmotic Pressure on DAP213

The point of time in vitrification was confirmed when osmotic pressure influences the survival rate of cells. First, three storage solutions (DAP-A, DAP-B, DAP-C) different in osmotic pressure were prepared according to the compositions described above. In this place, DAP-A was prepared by replacing the basal medium of DAP213 with PBS so that DAP-A has an osmotic pressure almost equal to that of DAP213. DAP-B was prepared in consideration of the dilution of the solution with the cell membrane permeable substance so that the final concentration of the salt is equal to the physiological environment. DAP-C was prepared so that the final concentration of the salt is twice the physiological environment. Mouse ES cells were suspended in DAP-A to C, and without vitrification the survival rate was measured after 90 seconds by diluting with PBS.

Specifically, experiment was conducted as follows:

· Mouse ES cells were cultured according to the ordinary method.
· The mouse ES cells recovered were counted, divided into $10^6$ cell portions in 1.5 ml tubes, and centrifuged at 1500 rpm for 5 minutes.
· The supernatant was removed, and the cells were suspended in 200 $\mu$l of PBS and 3 DAPs, respectively.
· The cells were left standing on ice.
· PBS (1800 $\mu$l) preliminarily heated to 37°C was added for suspension.
· The viable cells were counted by the trypan blue staining.
· On counting the cells, the life or death of cells was judged from the index of the selective excretive ability of the viable cells to trypan blue and the survival rate was calculated.

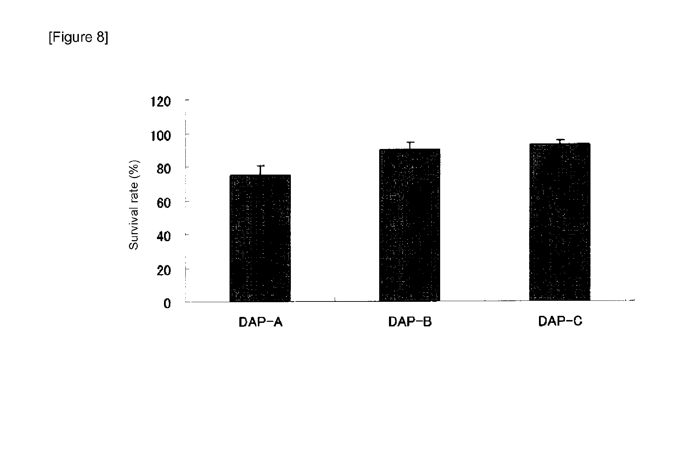

The results were as shown in Fig. 8.

In DAP-B and DAP-C, virtually all the cells were alive, in DAP-A, the viable cells were decreased by about 20%. It was shown from this result that the survival rate of cells is lowered on melting and dilution after vitrification in the storage solution with low osmotic pressure. In this connection, dead cells, which are positive to trypan blue, were scarcely observed in DAP-A in spite of the decrease of viable cells. It was suggested from this result that the dead cells were seriously damaged in the cell membrane.

As any one of DMSO, EG and PG used in the vitrification medium can be able to permeate the cell membrane, the volume of the cells at equilibrium is determined by the solutes other than these cell membrane permeable substances such as salts, sugars, amino acids and proteins. The time required for the equilibration depends on the sizes of the cells and the diffusion coefficients of permeable substances and is said to be about 1 to 5 minutes. In this test, vitrification is conducted after the cells are suspended in the storage solution and left standing for 60 seconds, and the mouse ES cells with relatively small size is believed to have completed the equilibration at this point of time. It was shown from the result of this test that the osmotic pressure of the vitrification medium influences the preservation efficiency, from which was shown the relationship between the preservation efficiency and the cell volume.

When the cells vitrified with DAP213 were counted after staining with trypan blue, it is often observed that the total number of viable cells and dead cells has been largely decreased from the original number of cells. Irrespective of this observation, the dead cells positive to trypan blue are extremely few. It was considered from this result that the survival rate on vitrificating the cells with DAP213 is lowered mainly by the cell burst due to the excessive expansion of the cells.

It was believed that the improvement of the survival rate by making the vitrification medium hypertonic is attributed to the control of the amount of the membrane permeable substance within the cells, which can withstand the influx of water into the cells on melting or dilution. It was also suggested that the survival rate of the cells vitrified after leaving at rest exceeded 90% even with the cell membrane permeable substance in high concentrations and thus the cytotoxicity of the cell membrane permeable substance, which has hitherto been said serious, had almost no influence.

As is apparent from above, the preservation efficiency of the vitrification medium was greatly influenced by osmotic pressure, and thus the survival rate was substantially improved by making the storage solution hypertonic.

EXAMPLE 7:

Materials:

Cell Membrane Non-Permeable Substance:

· Dimethyl sulfoxide (DMSO)
· Ethylene glycol (EG)
· Propylene glycol (PG)

Used Cells:

· Common marmoset ES cell line strain CMES40 (available from Riken Cell Bank)
· Feeder cell line, strain STO (available from Riken Cell Bank)

Composition of Culture Mediums used:

· KnockOut DMEM (manufactured by GIBCO)
· 20% Knockout Serum Replacement (manufactured by GIBCO)
· 0.1 mM Non-essential amino acids (manufactured by GIBCO)
· 0.1 mM 2-mercaptoethanol (manufactured by GIBCO)
· 2 mM L-glutamine (manufactured by GIBCO)
· 4 ng/ml bFGF (manufactured by Wako Pure Chemical Industries, Ltd.)

Vitrification Medium:

[Table 4]

|  | EG | PG | DMSO | Trehalose (*) | 10xPBS | Milli-Q | Total |
|---|---|---|---|---|---|---|---|
| DPT373 | 0 | 280 | 120 | 300 | 100 | 200 | 1000 |

(continued)

|  | EG | PG | DMSO | Trehalose (*) | 10xPBS | Milli-Q | Total |
|------|-----|----|------|---------------|--------|---------|-------|
| ET43 | 400 | 0 | 0 | 300 | 100 | 200 | 1000 |

(*) Trehalose is dissolved in Milli-Q so that the concentration is 1 M.

[Table 5]

| Osmotic pressure (mOsm) | EG | Trehalose (*) | 10xPBS | Milli-Q | Total |
|------------------------:|-----|---------------|--------|---------|-------|
| 600 | 400 | 300 | 100 | 200 | 1000 |
| 800 | 400 | 300 | 166 | 134 | 1000 |
| 1000 | 400 | 300 | 233 | 67 | 1000 |
| 1200 | 400 | 300 | 300 | 0 | 1000 |

(*) Trehalose is dissolved in Milli-Q so that the concentration is 1 M.

[Table 6]

|  | EG | Trehalose (*) | 10xPBS | Total |
|------|-----|---------------|--------|-------|
| ET45 | 400 | 500 | 100 | 1000 |

(*) Trehalose is dissolved in Milli-Q so that the concentration is 1 M.

Alkaline Phosphatase (ALP) Staining:

· Leukocyte Alkaline phosphatase Kit (sigma 86R)
· Acetone
· 37% Formaldehyde
· Milli-Q

(7-1) Preservation Effect on Common Marmoset ES Cells

CMES cells were vitrified in a storage solution containing DMSO+PG or EG as a cell membrane permeable substance and trehalose as a vitrification promoting substance, and the survival rate after melting was examined. In the evaluation with the mouse ES cells, the combination of DMSO and PG as the cell membrane permeable substance is most excellent, but there has been described the differentiation inducibility of DMSO to the ES cells, and thus EG having the second highest survival rate in the mouse ES cells was also evaluated.

Specifically, experiment was conducted as follows:

· CMES cells were cultured with STO cells as a feeder according to the ordinary method.
· On the previous day of test, STO cells were seeded in $\varphi$35 mm dishes in an amount of $10^6$ cell/dish according to the ordinary method.
· On the day of test, the culture medium for STO cells was replaced with 1.5 ml of CMES cell maintenance medium for incubation.
· The CMES cells were recovered according to the ordinary method and dispensed from a $\varphi$90 mm dish into 6 to 9 1.5 ml microtubes.
· The tubes were centrifuged at 1500 rpm for 5 minutes and left standing on ice.
· In the control section (not frozen), the cells suspended into 1 ml of the culture medium were seeded on a 450 $\mu$l dish to which STO had preliminarily been adhered.
· In test section, supernatants were removed and 200 $\mu$l of the vitrification medium was added and suspended.

· After suspension, DAP213 and the test fluid were dipped into liquid nitrogen after 15 and 60 seconds and 60 seconds, respectively, and vitrified.

· The tubes are left standing for 5 minutes or more to stabilize the temperature.

· The tubes were taken out from liquid nitrogen, and 1800 $\mu$l of the culture medium preliminarily heated to 37°C was added and rapidly suspended for melting.

· After melting, 900 $\mu$l portions of the suspension were dispensed into 1.5 ml microtubes and centrifuged at 1500 rpm for 5 minutes to remove supernatants.

· The cells were suspended in 450 $\mu$l of the culture medium, and the total amount of the cells was seeded on the $\varphi$35 mm dish described above.

· The culture medium was replaced every day after 2 days of the seeding, the cells were stained with ALP after 3 or 4 days depending on the condition of the colonies, and positive colonies were counted.

· The ratio of the positive colonies in the test section to the control section was calculated as the take rate.

It was revealed that high preservation efficiency was shown in the case of using EG alone as the cell membrane permeable substance to the CMES cells.

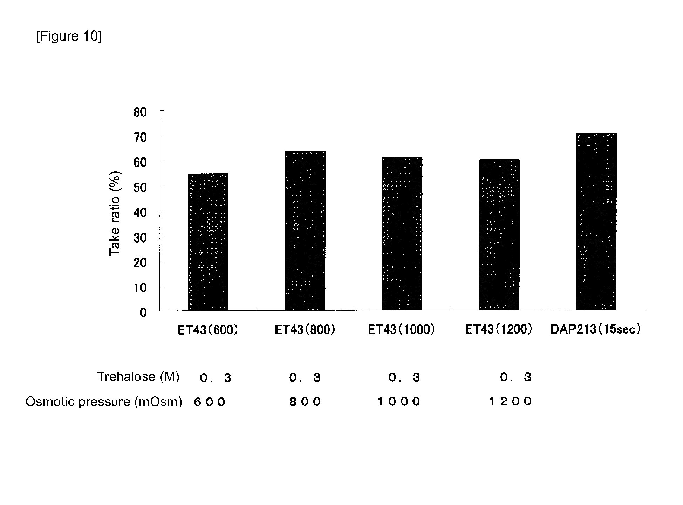

Next, osmotic pressure was adjusted in order to examine the influence on the preservation efficiency. The results were as shown in Fig. 10.

The take rate was excellent when the osmotic pressure was adjusted to 800 mOsm or more by combining sugars and salts, but further improvement of the preservation efficiency was not observed even if the osmotic pressure was increased to more than 800 mOsm.

Based on these results, with use of 40% EG as the cell membrane permeable substance and trehalose as the vitrification promoting substance, the total osmotic pressure of the salt and trehalose was adjusted to 800 mOsm (ET45), and the take rate of CMES subjected to suspension followed by vitrification and melting after 15 seconds to 60 seconds was compared with that of DAP213.

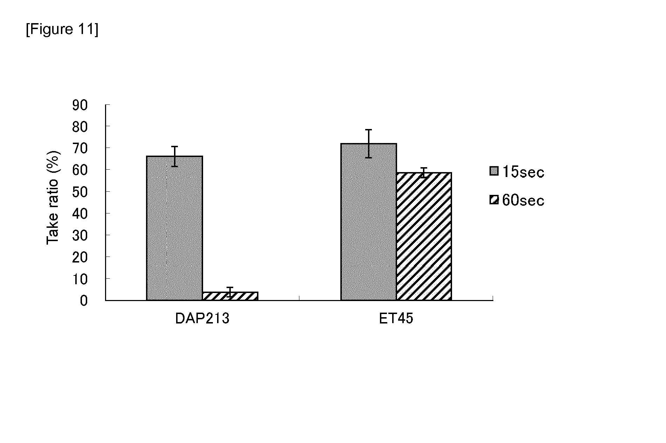

The results were as shown in Fig. 11.

ET45 exhibited a higher take rate than DAP213 in either case of vitrification within 15 seconds after suspending the CMES cells or vitrification after 60 seconds.

EXAMPLE 8:

Examination of Ice Crystal Growth Inhibitor

Examination of the ice crystal growth inhibitor was conducted by comparing the times required for melting the crystallized vitrificated storage solutions. That is, when crystal lumps having the same volume are melted, a polycrystalline substance having a larger total surface area is melted more quickly. It is believed that when the growth of ice crystals is suppressed on the phase transition to crystalline state, the melting time is shortened probably due to the formation of many minute ice crystals.

Materials:

Cell Membrane Permeable Substance:

· Ethylene glycol (EG)

Ice Crystal Growth Inhibitor:

· Sericin

(Comparative Example)

· Bovine serum albumin (BSA)
· Trehalose

· Sucrose

Storage Solution used:

| | | |
|---|---|---|
| · EG | | 400 µl |
| · 10% by weight of various ice crystal growth inhibitor | | 100 to 500 µl |
| · Milli-Q | | 100 to 500 µl |
| | Total | 1000 µl |

Specifically, experiment was conducted according to the following procedure.

· The test fluid prepared was dispensed in a portion of 200 µl into 1.5 ml microtubes.
· Dewar vessel was charged with liquid nitrogen, and a timer was provided.
· The microtube was picked up with tweezers and dipped into liquid nitrogen for 40 seconds for vitrification.
· The microtube was pulled out from liquid nitrogen, and the time required for complete disappearance of ice within the tube was measured.

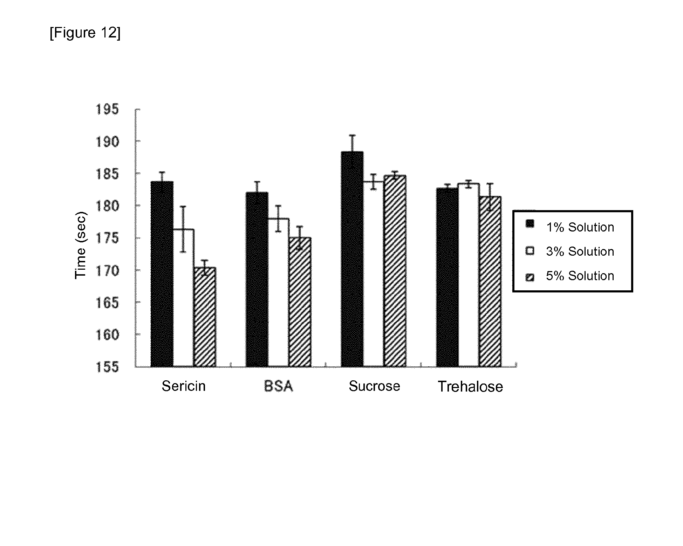

The result was as shown in Fig. 12.
The time required for the once vitrificated storage solution to be melted completely was shortened depending on the concentration of sericin. While albumin also exhibited an effect similar to that of sericin, its activity remained unsatisfactory. In addition, sucrose and trehalose generally used as a vitrification promoting substance exhibited no reduction of melting time. It was confirmed from no reduction of melting time in low molecular weight saccharides that this effect is not induced from mere depression of freezing point.
It was suggested from these results that sericin suppresses the growth of ice crystals.

**Claims**

1. A vitrification medium for cells comprising a cell membrane permeable substance and a cell membrane non-permeable substance, in which
the content of the cell membrane permeable substance is in the range of 30 to 50% by volume, and
the osmotic pressure generated in association with the cell membrane non-permeable substance, which is a fraction of the total osmotic pressure on the cell membrane on suspending the cells in the vitrification medium, is in the range of 280 mOsm or more.

2. A vitrification medium for cells according to claim 1, wherein the cell membrane permeable substance is one or more selected from the group consisting of ethylene glycol (EG), propylene glycol (PG), 1,3-propanediol (1,3-PD), butylene glycol (BG), isoprene glycol (IPG), dipropylene glycol (DPG), glycerin and dimethyl sulfoxide (DMSO).

3. A vitrification medium for cells according to claims 1 or 2, wherein the cell membrane permeable substance is one or two selected from the group consisting of ethylene glycol (EG), propylene glycol (PG) and dimethyl sulfoxide (DMSO).

4. A vitrification medium for cells according to any one of claims 1 to 3, wherein the cell membrane non-permeable substance is one or more selected from the group consisting of sodium chloride, potassium chloride, disodium hydrogen phosphate, potassium dihydrogen phosphate, monosaccharides, disaccharides, trisaccharides, polysaccharides, sugar alcohols, ficolls, polyethylene glycol, polyvinyl alcohol and polyvinylpyrrolidone.

5. A vitrification medium for cells according to any one of claims 1 to 4, wherein the cell membrane non-permeable substance comprises a physiological environment adjusting substance selected from the group consisting of sodium chloride, potassium chloride, disodium hydrogen phosphate and potassium dihydrogen phosphate and a vitrification promoting substance selected from the group consisting of trehalose and sucrose.

6. A vitrification medium for cells according to any one of claims 1 to 5, wherein the cell membrane non-permeable substance comprises trehalose having a concentration of 0.2 M to 1 M in the vitrification medium.

7.  A vitrification medium for cells according to any one of claims 1 to 6, which comprises further an ice crystal growth inhibitor selected from the group consisting of an antifreeze protein and sericin.

8.  A vitrification medium for cells according to any one of claims 1 to 7, which is used for the vitrification preservation of pluripotent stem cells.

9.  A vitrification medium for cells according to any one of claims 1 to 8, which is used for the vitrification preservation of primate ES cells or primate iPS cells.

10. A vitrification method for cells which comprises suspending cells in the vitrification medium for cells according to any one of claims 1 to 9 followed by rapidly cooling the vitrification medium containing the cells with liquid nitrogen for vitrification, in which

the osmotic pressure generated in association with the cell membrane non-permeable substance, which is a fraction of the total osmotic pressure on the cell membrane on suspending the cells in the vitrification medium, is in the range of 280 mOsm or more.

[Figure 1]

VOLUME CHANGES BEFORE VITRIFICATION

[Figure 2]

AMOUNT CHANGES OF THE CELL MEMBRANE PERMEABLE SUBSTANCE

[Figure 3]

VOLUME CHANGES ON MELTING

[Figure 4]

SURVIVAL RATE AFTER VITRIFICATION

[Figure 5]

[Figure 6]

[Figure 7]

| | Physiological salt (isotonic) | High concentration salt (hypertonic) | Physiological salt + trehalose (hypertonic) | No salt + trehalose (hypertonic) |
|---|---|---|---|---|
| Trehalose (M) | — | — | 0.3 | 0.6 |
| Osmotic pressure (mOsm) | 300 | 600 | 600 | 600 |

[Figure 8]

[Figure 9]

※ No measurement at 15 seconds.

[Figure 10]

| | ET43(600) | ET43(800) | ET43(1000) | ET43(1200) |
|---|---|---|---|---|
| Trehalose (M) | 0. 3 | 0. 3 | 0. 3 | 0. 3 |
| Osmotic pressure (mOsm) | 6 0 0 | 8 0 0 | 1 0 0 0 | 1 2 0 0 |

[Figure 11]

[Figure 12]

## INTERNATIONAL SEARCH REPORT

| | International application No. |
|---|---|
| | PCT/JP2011/076711 |

| A. CLASSIFICATION OF SUBJECT MATTER |
|---|
| *C12N5/07*(2010.01)i |

According to International Patent Classification (IPC) or to both national classification and IPC

| B. FIELDS SEARCHED |
|---|
| Minimum documentation searched (classification system followed by classification symbols) |
| C12N1/00-7/08 |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII), BIOSIS/MEDLINE/WPIDS(STN)

| C. DOCUMENTS CONSIDERED TO BE RELEVANT | | |
|---|---|---|
| Category* | Citation of document, with indication, where appropriate, of the relevant passages | Relevant to claim No. |
| X/Y | WO 2005/045007 A1 (ReproCELL Inc.), 19 May 2005 (19.05.2005), (Family: none) | 1-6,8-10/7 |
| X/Y | Tamotsu YAMASHIRO et al., "Ushi Hai no Jutairitsu Kojo Shiken", Okinawa Prefectural Livestock and Grassland Research Center Shiken Kenkyu Hokoku no.47, 15 October 2010 (15.10. 2010), pages 1 to 6 | 1-5,8/6,7,9, 10 |
| Y | JP 2006-115837 A (Seiren Co., Ltd.), 11 May 2006 (11.05.2006), & US 2009/0197331 A1 & EP 1820847 A1 & WO 2006/033429 A1 | 1-10 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 30 January, 2012 (30.01.12) | 07 February, 2012 (07.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

| INTERNATIONAL SEARCH REPORT | International application No. |
|---|---|
| | PCT/JP2011/076711 |

C (Continuation). DOCUMENTS CONSIDERED TO BE RELEVANT

| Category* | Citation of document, with indication, where appropriate, of the relevant passages | Relevant to claim No. |
|---|---|---|
| Y | ODA,K., et al., "Osmotic shock of fertilized mouse ova" J.Reprod.Fert., 1992, Vol.95, p.737-747 | 1-10 |
| Y | KATKOV,I.I. & POGORELOV,A.G. "Influence of exposure to vitrification solutions on 2-cell mouse embryos: II. Osmotic effects or chemical toxicity?" CryoLetters, 2007, Vol.28, No.6, p.409-427 | 1-10 |
| Y | LIN,L., et al., "Osmotic stress induced by sodium chloride, sucrose or trehalose improves cryotolerance and developmental competence of porcine oocytes" Reprod.Fertil.Develop., 2009, Vol.21, p.338-344 | 1-10 |
| Y | SONGSASEN,N., et al., "Permeability characteristics and osmotic sensitivity of rhesus monkey(Macaca mulatta) oocytes" Human Reprod., 2002, Vol.17, no.7, p.1875-1884 | 1-10 |
| A | NAKASHIMA,A., et al., "Optimization of a novel nylon mesh container for human embryo ultrarapid vitrification" Fertil.Steril., 2010.05.01, Vol.93, No.7, p.2405-2410 | 1-10 |
| A | Koji MISUMI et al., "Dobutsu Yurai Busshitsu o Fukumanai Glass-ka Hozon-yo Baieki ni yoru Buta Hai no Glass-ka Hozon", Dai 110 Kai The Japanese Society of Zootechnical Science Annual Meeting abstracts, 27 March 2009 (27.03.2009), page 79 | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010259525 A
- WO 2005045007 A
- WO 2007058308 A
- JP 2001502664 A

- WO 9809514 A
- JP 2001517204 A
- WO 9745010 A
- JP 3694730 B

**Non-patent literature cited in the description**

- **FUJIOKA T ; YASUCHIKA K ; NAKAMURA Y ; NAKATSUJI N ; SUEMORI H.** A simple and efficient cryopreservation method for primate embryonic stem cells. *Int J Dev Biol,* 2004, vol. 48, 1149-54
- *Protocol for maintaining and culturing human pluripotent stem cell,* 2010

- **SASAKI E ; HANAZAWA K ; KURITA R ; AKATSUKA A ; YOSHIZAKI T ; ISHII H ; TANIOKA Y ; OHNISHI Y ; SUEMIZU H ; SUGAWARA A.** *Stem Cells.,* October 2005, vol. 23 (9), 1304-13